# EUROPEAN PATENT SPECIFICATION

(11) **EP 2 907 523 B1**
(45) Date of publication and mention of the grant of the patent: **28.03.2018**
(21) Application number: 14195149.1
(22) Date of filing: 29.01.2010
(51) Int. Cl.: A61K 39/118, A61P 31/04, A61P 37/04, A61K 39/00

(54) **Compositions comprising chlamydia antigens**
Zusammensetzungen mit Chlamydia-Antigenen
Compositions comprenant des antigènes de chlamydia

(30) Priority: 29.01.2009 US 202104 P; 22.04.2009 US 202943 P
(43) Date of publication of application: 19.08.2015
(62) Divisional of application: 10735471.4
(73) Proprietor: British Columbia Cancer Agency Branch, Vancouver, British Columbia V5Z 4E6 (CA)
(72) Inventor: Brunham, Robert C, Vancouver British Columbia V5Z 4R4 (CA); Foster, Leonard J, Vancouver British Columbia V6T 1Z4 (CA)
(74) Representative: Barker Brettell LLP

(56) References cited:
- WO-A2-02/062380
- WO-A2-2006/045308
- WO-A2-2006/104890
- KARUNAKARAN, K. ET AL.: "Immunoproteomic discovery of novel T cell antigens from the obligate intracellular pathogen Chlamydia", JOURNAL IMMUNOLOGY, vol. 180, 2008, pages 2459-2465, XP002694535,
- HANSEN ET AL.: "Liposome delivery of Chlamydia muridarum major outer membrane protein primes a Thl response that protects against chlamydial infection in a mouse model", JOURNAL INFECTIOUS DISEASES, vol. 198, September 2008 (2008-09), pages 758-767, XP002694536,

### SEQUENCE LISTING

<110> British Columbia Cancer Agency
<120> Compositions Comprising Chlamydia Antigens
<130> JA60383P.EPPD1
<140>
   <141>
<150> EP10735471.4
   <151> 2010-01-29
<150> PCT/CA2010/000135
   <151> 2010-01-29
<150> 61/202,104
   <151> 2009-01-29
<150> 61/202,943
   <151> 2009-04-22
<160> 48
<170> PatentIn version 3.5
<210> 1
<400> 1
   000
<210> 2
<400> 2
   000
<210> 3
<400> 3
   000
<210> 4
<400> 4
   000
<210> 5
<400> 5
   000
<210> 6
<400> 6
   000
<210> 7
<400> 7
   000
<210> 8
<400> 8
   000
<210> 9
<400> 9
   000
<210> 10
   <211> 17
   <212> PRT
   <213> Artificial sequence
<220>
   <223> Ribosomal protein L/Rplf
<400> 10
   Gly Asn Glu Val Phe Val Ser Pro Ala Ala His Ile Ile Asp Arg Pro
   1               5                   10                  15
   Gly
<210> 11
   <211> 18
   <212> PRT
   <213> Artificial sequence
<220>
   <223> ACP reductase
<400> 11
   Ser Pro Gly Gln Thr Asn Tyr Ala Ala Ala Lys Ala Gly Ile Ile Gly
   1 5 10 15
   Phe Ser
<210> 12
   <211> 16
   <212> PRT
   <213> Artificial sequence
<220>
   <223> Anti-anti-sigma factor
<400> 12
   Lys Leu Asp Gly Val Ser Ser Pro Ala Val Gln Glu Ser Ile Ser Glu
   1 5 10 15
<210> 13
   <211> 17
   <212> PRT
   <213> Artificial sequence
<220>
   <223> Polymorphic membrane protein G
<400> 13
   Ala Ser Pro Ile Tyr Val Asp Pro Ala Ala Ala Gly Gly Gln Pro Pro
   1 5 10 15
   Ala
<210> 14
   <211> 14
   <212> PRT
   <213> Artificial sequence
<220>
   <223> Hypothetical protein
<400> 14
   Asp Leu Asn Val Thr Gly Pro Lys Ile Gln Thr Asp Val Asp
   1 5 10
<210> 15
   <211> 15
   <212> PRT
   <213> Artificial sequence
<220>
   <223> ATP-dependent Clp protease proteolytic subunit
<400> 15
   Ile Gly Gln Glu Ile Thr Glu Pro Leu Ala Asn Thr Val Ile Ala
   1               5                   10                  15
<210> 16
   <211> 16
   <212> PRT
   <213> Artificial sequence
<220>
   <223> Polymorphic membrane protein F
<400> 16
   Ala Phe His Leu Phe Ala Ser Pro Ala Ala Asn Tyr Ile His Thr Gly
   1               5                   10                  15
<210> 17
   <211> 15
   <212> PRT
   <213> Artificial sequence
<220>
   <223> Glyceraldehyde 3-phosphate dehydrogenase
<400> 17
   Met Thr Thr Val His Ala Ala Thr Ala Thr Gln Ser Val Val Asp
   1               5                   10                  15
<210> 18
<400> 18
   000
<210> 19
   <211> 8
   <212> PRT
   <213> Chlamydia trachomatis
<400> 19
   Ser Ser Leu Phe Leu Val Lys Leu
   1               5
<210> 20
   <211> 14
   <212> PRT
   <213> Chlamydia trachomatis
<400> 20
   Gly Asn Glu Val Phe Val Ser Pro Ala Ala His Ile Ile Asp
   1               5                   10
<210> 21
   <211> 17
   <212> PRT
   <213> Chlamydia trachomatis
<400> 21
   Gly Asn Glu Val Phe Val Ser Pro Ala Ala His Ile Ile Asp Arg Pro
   1               5                   10                  15
   Gly
<210> 22
   <211> 18
   <212> PRT
   <213> Chlamydia trachomatis
<400> 22
   Lys Gly Asn Glu Val Phe Val Ser Pro Ala Ala His Ile Ile Asp Arg
   1               5                   10                  15
   Pro Gly
<210> 23
   <211> 15
   <212> PRT
   <213> Chlamydia trachomatis
<400> 23
   Glu Val Phe Val Ser Pro Ala Ala His Ile Ile Asp Arg Pro Gly
   1               5                   10                  15
<210> 24
   <211> 16
   <212> PRT
   <213> Chlamydia trachomatis
<400> 24
   Ser Pro Gly Gln Thr Asn Tyr Ala Ala Ala Lys Ala Gly Ile Ile Gly
   1               5                   10                  15
<210> 25
   <211> 18
   <212> PRT
   <213> Chlamydia trachomatis
<400> 25
   Ser Pro Gly Gln Thr Asn Tyr Ala Ala Ala Lys Ala Gly Ile Ile Gly
   1               5                   10                  15
   Phe Ser
<210> 26
   <211> 16
   <212> PRT
   <213> Chlamydia trachomatis
<400> 26
   Lys Leu Asp Gly Val Ser Ser Pro Ala Val Gln Glu Ser Ile Ser Glu
   1 5 10 15
<210> 27
   <211> 16
   <212> PRT
   <213> Chlamydia trachomatis
<400> 27
   Ser Pro Ile Tyr Val Asp Pro Ala Ala Ala Gly Gly Gln Pro Pro Ala
   1 5 10 15
<210> 28
   <211> 17
   <212> PRT
   <213> Chlamydia trachomatis
<400> 28
   Ala Ser Pro Ile Tyr Val Asp Pro Ala Ala Ala Gly Gly Gln Pro Pro
   1 5 10 15
   Ala
<210> 29
   <211> 14
   <212> PRT
   <213> Chlamydia trachomatis
<400> 29
   Asp Leu Asn Val Thr Gly Pro Lys Ile Gln Thr Asp Val Asp
   1 5 10
<210> 30
   <211> 15
   <212> PRT
   <213> Chlamydia trachomatis
<400> 30
   Ile Gly Gln Glu Ile Thr Glu Pro Leu Ala Asn Thr Val Ile Ala
   1 5 10 15
<210> 31
   <211> 16
   <212> PRT
   <213> Chlamydia trachomatis
<400> 31
   Phe His Leu Phe Ala Ser Pro Ala Ala Asn Tyr Ile His Thr Gly Pro
   1 5 10 15
<210> 32
   <211> 15
   <212> PRT
   <213> Chlamydia trachomatis
<400> 32
   Met Thr Thr Val His Ala Ala Thr Ala Thr Gln Ser Val Val Asp
   1               5                   10                  15
<210> 33
<400> 33
   000
<210> 34
<400> 34
   000
<210> 35
<400> 35
   000
<210> 36
<400> 36
   000
<210> 37
<400> 37
   000
<210> 38
<400> 38
   000
<210> 39
<400> 39
   000
<210> 40
<400> 40
   000
<210> 41
<400> 41
   000
<210> 42
   <211> 487
   <212> PRT
   <213> Chlamydia trachomatis
<400> 42
   Tyr Ala Ala Glu Ile Met Ile Pro Gln Gly Ile Tyr Asp Gly Glu Thr
   1               5                   10                  15
   Leu Thr Val Ser Phe Pro Tyr Thr Val Ile Gly Asp Pro Ser Gly Thr
               20                  25                  30
   Thr Val Phe Ser Ala Gly Glu Leu Thr Leu Lys Asn Leu Asp Asn Ser
           35                  40                  45
   Ile Ala Ala Leu Pro Leu Ser Cys Phe Gly Asn Leu Leu Gly Ser Phe
       50                  55                  60
   Thr Val Leu Gly Arg Gly His Ser Leu Thr Phe Glu Asn Ile Arg Thr
   65                  70                  75                  80
   Ser Thr Asn Gly Ala Ala Leu Ser Asp Ser Ala Asn Ser Gly Leu Phe
                   85                  90                  95
   Thr Ile Glu Gly Phe Lys Glu Leu Ser Phe Ser Asn Cys Asn Ser Leu
               100                 105                 110
   Leu Ala Val Leu Pro Ala Ala Thr Thr Asn Asn Gly Ser Gln Thr Pro
           115                 120                 125
   Thr Thr Thr Ser Thr Pro Ser Asn Gly Thr Ile Tyr Ser Lys Thr Asp
       130                 135                 140
   Leu Leu Leu Leu Asn Asn Glu Lys Phe Ser Phe Tyr Ser Asn Leu Val
   145                 150                 155                 160
   Ser Gly Asp Gly Gly Ala Ile Asp Ala Lys Ser Leu Thr Val Gln Gly
                   165                 170                 175
   Ile Ser Lys Leu Cys Val Phe Gln Glu Asn Thr Ala Gln Ala Asp Gly
               180                 185                 190
   Gly Ala Cys Gln Val Val Thr Ser Phe Ser Ala Met Ala Asn Glu Ala
           195                 200                 205
   Pro Ile Ala Phe Ile Ala Asn Val Ala Gly Val Arg Gly Gly Gly Ile
       210                 215                 220
   Ala Ala Val Gln Asp Gly Gln Gln Gly Val Ser Ser Ser Thr Ser Thr
   225                 230                 235                 240
   Glu Asp Pro Val Val Ser Phe Ser Arg Asn Thr Ala Val Glu Phe Asp
                   245                 250                 255
   Gly Asn Val Ala Arg Val Gly Gly Gly Ile Tyr Ser Tyr Gly Asn Val
               260                 265                 270
   Ala Phe Leu Asn Asn Gly Lys Thr Leu Phe Leu Asn Asn Val Ala Ser
           275                 280                 285
   Pro Val Tyr Ile Ala Ala Glu Gln Pro Thr Asn Gly Gln Ala Ser Asn
       290                 295                 300
   Thr Ser Asp Asn Tyr Gly Asp Gly Gly Ala Ile Phe Cys Lys Asn Gly
   305                 310                 315                 320
   Ala Gln Ala Ala Gly Ser Asn Asn Ser Gly Ser Val Ser Phe Asp Gly
                   325                 330                 335
   Glu Gly Val Val Phe Phe Ser Ser Asn Val Ala Ala Gly Lys Gly Gly
               340                 345                 350
   Ala Ile Tyr Ala Lys Lys Leu Ser Val Ala Asn Cys Gly Pro Val Gln
           355                 360                 365
   Phe Leu Gly Asn Ile Ala Asn Asp Gly Gly Ala Ile Tyr Leu Gly Glu
       370                 375                 380
   Ser Gly Glu Leu Ser Leu Ser Ala Asp Tyr Gly Asp Ile Ile Phe Asp
   385                 390                 395                 400
   Gly Asn Leu Lys Arg Thr Ala Lys Glu Asn Ala Ala Asp Val Asn Gly
                   405                 410                 415
   Val Thr Val Ser Ser Gln Ala Ile Ser Met Gly Ser Gly Gly Lys Ile
               420                 425                 430
   Thr Thr Leu Arg Ala Lys Ala Gly His Gln Ile Leu Phe Asn Asp Pro
           435                 440                 445
   Ile Glu Met Ala Asn Gly Asn Asn Gln Pro Ala Gln Ser Ser Glu Pro
       450                 455                 460
   Leu Lys Ile Asn Asp Gly Glu Gly Tyr Thr Gly Asp Ile Val Phe Ala
   465                 470                 475                 480
   Asn Gly Asn Ser Thr Leu Tyr
                   485
<210> 43
   <211> 559
   <212> PRT
   <213> Chlamydia trachomatis
<400> 43
   Glu Thr Asp Thr Leu Gln Phe Arg Arg Phe Thr Phe Ser Asp Arg Glu
   1 5 10 15
   Ile Gln Phe Val Leu Asp Pro Ala Ser Leu Ile Thr Ala Gln Asn Ile
   20 25 30
   Val Leu Ser Asn Leu Gln Ser Asn Gly Thr Gly Ala Cys Thr Ile Ser
   35 40 45
   Gly Asn Thr Gln Thr Gln Ile Phe Ser Asn Ser Val Asn Thr Thr Ala
   50 55 60
   Asp Ser Gly Gly Ala Phe Asp Met Val Thr Thr Ser Phe Thr Ala Ser
   65 70 75 80
   Asp Asn Ala Asn Leu Leu Phe Cys Asn Asn Tyr Cys Thr His Asn Lys
   85 90 95
   Gly Gly Gly Ala Ile Arg Ser Gly Gly Pro Ile Arg Phe Leu Asn Asn
   100 105 110
   Gln Asp Val Leu Phe Tyr Asn Asn Ile Ser Ala Gly Ala Lys Tyr Val
   115 120 125
   Gly Thr Gly Asp His Asn Glu Lys Asn Arg Gly Gly Ala Leu Tyr Ala
   130 135 140
   Thr Thr Ile Thr Leu Thr Gly Asn Arg Thr Leu Ala Phe Ile Asn Asn
   145 150 155 160
   Met Ser Gly Asp Cys Gly Gly Ala Ile Ser Ala Asp Thr Gln Ile Ser
   165 170 175
   Ile Thr Asp Thr Val Lys Gly Ile Leu Phe Glu Asn Asn His Thr Leu
   180 185 190
   Asn His Ile Pro Tyr Thr Gln Ala Glu Asn Met Ala Arg Gly Gly Ala
   195 200 205
   Ile Cys Ser Arg Arg Asp Leu Cys Ser Ile Ser Asn Asn Ser Gly Pro
       210                 215                 220
   Ile Val Phe Asn Tyr Asn Gln Gly Gly Lys Gly Gly Ala Ile Ser Ala
   225                 230                 235                 240
   Thr Arg Cys Val Ile Asp Asn Asn Lys Glu Arg Ile Ile Phe Ser Asn
                   245                 250                 255
   Asn Ser Ser Leu Gly Trp Ser Gln Ser Ser Ser Ala Ser Asn Gly Gly
               260                 265                 270
   Ala Ile Gln Thr Thr Gln Gly Phe Thr Leu Arg Asn Asn Lys Gly Ser
           275                 280                 285
   Ile Tyr Phe Asp Ser Asn Thr Ala Thr His Ala Gly Gly Ala Ile Asn
       290                 295                 300
   Cys Gly Tyr Ile Asp Ile Arg Asp Asn Gly Pro Val Tyr Phe Leu Asn
   305                 310                 315                 320
   Asn Ser Ala Ala Trp Gly Ala Ala Phe Asn Leu Ser Lys Pro Arg Ser
                   325                 330                 335
   Ala Thr Asn Tyr Ile His Thr Gly Thr Gly Asp Ile Val Phe Asn Asn
               340                 345                 350
   Asn Val Val Phe Thr Leu Asp Gly Asn Leu Leu Gly Lys Arg Lys Leu
           355                 360                 365
   Phe His Ile Asn Asn Asn Glu Ile Thr Pro Tyr Thr Leu Ser Leu Gly
       370                 375                 380
   Ala Lys Lys Asp Thr Arg Ile Tyr Phe Tyr Asp Leu Phe Gln Trp Glu
   385                 390                 395                 400
   Arg Val Lys Glu Asn Thr Ser Asn Asn Pro Pro Ser Pro Thr Ser Arg
                   405                 410                 415
   Asn Thr Ile Thr Val Asn Pro Glu Thr Glu Phe Ser Gly Ala Val Val
               420                 425                 430
   Phe Ser Tyr Asn Gln Met Ser Ser Asp Ile Arg Thr Leu Met Gly Lys
           435                 440                 445
   Glu His Asn Tyr Ile Lys Glu Ala Pro Thr Thr Leu Lys Phe Gly Thr
       450                 455                 460
   Leu Ala Ile Glu Asp Asp Ala Glu Leu Glu Ile Phe Asn Ile Pro Phe
   465                 470                 475                 480
   Thr Gln Asn Pro Thr Ser Leu Leu Ala Leu Gly Ser Gly Ala Thr Leu
                   485                 490                 495
   Thr Val Gly Lys His Gly Lys Leu Asn Ile Thr Asn Leu Gly Val Ile
               500                 505                 510
   Leu Pro Ile Ile Leu Lys Glu Gly Lys Ser Pro Pro Cys Ile Arg Val
           515                 520                 525
   Asn Pro Gln Asp Met Thr Gln Asn Thr Gly Thr Gly Gln Thr Pro Ser
       530                 535                 540
   Ser Thr Ser Ser Ile Ser Thr Pro Met Ile Ile Phe Asn Gly Arg
   545 550 555
<210> 44
   <211> 371
   <212> PRT
   <213> Chlamydia trachomatis
<400> 44
   Leu Pro Val Gly Asn Pro Ala Glu Pro Ser Leu Met Ile Asp Gly Ile
   1               5                   10                  15
   Leu Trp Glu Gly Phe Gly Gly Asp Pro Cys Asp Pro Cys Ala Thr Trp
               20                  25                  30
   Cys Asp Ala Ile Ser Met Arg Val Gly Tyr Tyr Gly Asp Phe Val Phe
           35                  40                  45
   Asp Arg Val Leu Lys Thr Asp Val Asn Lys Glu Phe Gln Met Gly Ala
       50                  55                  60
   Lys Pro Thr Thr Asp Thr Gly Asn Ser Ala Ala Pro Ser Thr Leu Thr
   65                  70                  75                  80
   Ala Arg Glu Asn Pro Ala Tyr Gly Arg His Met Gln Asp Ala Glu Met
                   85                  90                  95
   Phe Thr Asn Ala Ala Cys Met Ala Leu Asn Ile Trp Asp Arg Phe Asp
               100                 105                 110
   Val Phe Cys Thr Leu Gly Ala Thr Ser Gly Tyr Leu Lys Gly Asn Ser
           115                 120                 125
   Ala Ser Phe Asn Leu Val Gly Leu Phe Gly Asp Asn Glu Asn Gln Lys
       130                 135                 140
   Thr Val Lys Ala Glu Ser Val Pro Asn Met Ser Phe Asp Gln Ser Val
   145                 150                 155                 160
   Val Glu Leu Tyr Thr Asp Thr Thr Phe Ala Trp Ser Val Gly Ala Arg
                   165                 170                 175
   Ala Ala Leu Trp Glu Cys Gly Cys Ala Thr Leu Gly Ala Ser Phe Gln
               180                 185                 190
   Tyr Ala Gln Ser Lys Pro Lys Val Glu Glu Leu Asn Val Leu Cys Asn
           195                 200                 205
   Ala Ala Glu Phe Thr Ile Asn Lys Pro Lys Gly Tyr Val Gly Lys Glu
       210                 215                 220
   Phe Pro Leu Asp Leu Thr Ala Gly Thr Asp Ala Ala Thr Gly Thr Lys
   225                 230                 235                 240
   Asp Ala Ser Ile Asp Tyr His Glu Trp Gln Ala Ser Leu Ala Leu Ser
                   245                 250                 255
   Tyr Arg Leu Asn Met Phe Thr Pro Tyr Ile Gly Val Lys Trp Ser Arg
               260                 265                 270
   Ala Ser Phe Asp Ala Asp Thr Ile Arg Ile Ala Gln Pro Lys Ser Ala
               275                 280                 285
   Thr Ala Ile Phe Asp Thr Thr Thr Leu Asn Pro Thr Ile Ala Gly Ala
       290                 295                 300
   Gly Asp Val Lys Thr Gly Ala Glu Gly Gln Leu Gly Asp Thr Met Gln
   305                 310                 315                 320
   Ile Val Ser Leu Gln Leu Asn Lys Met Lys Ser Arg Lys Ser Cys Gly
                   325                 330                 335
   Ile Ala Val Gly Thr Thr Ile Val Asp Ala Asp Lys Tyr Ala Val Thr
               340                 345                 350
   Val Glu Thr Arg Leu Ile Asp Glu Arg Ala Ala His Val Asn Ala Gln
           355                 360                 365
   Phe Arg Phe
       370
<210> 45
   <211> 475
   <212> PRT
   <213> Chlamydia muridarum
<400> 45
   Ala Asp Ile Ser Met Pro Pro Gly Ile Tyr Asp Gly Thr Thr Leu Thr
   1               5                   10                  15
   Ala Pro Phe Pro Tyr Thr Val Ile Gly Asp Pro Arg Gly Thr Lys Val
               20                  25                  30
   Thr Ser Ser Gly Ser Leu Glu Leu Lys Asn Leu Asp Asn Ser Ile Ala
           35                  40                  45
   Thr Leu Pro Leu Ser Cys Phe Gly Asn Leu Leu Gly Asn Phe Thr Ile
       50                  55                  60
   Ala Gly Arg Gly His Ser Leu Val Phe Glu Asn Ile Arg Thr Ser Thr
   65                  70                  75                  80
   Asn Gly Ala Ala Leu Ser Asn His Ala Pro Ser Gly Leu Phe Val Ile
                   85                  90                  95
   Glu Ala Phe Asp Glu Leu Ser Leu Leu Asn Cys Asn Ser Leu Val Ser
               100                 105                 110
   Val Val Pro Gln Thr Gly Gly Thr Thr Thr Ser Val Pro Ser Asn Gly
           115                 120                 125
   Thr Ile Tyr Ser Arg Thr Asp Leu Val Leu Arg Asp Ile Lys Lys Val
       130                 135                 140
   Ser Phe Tyr Ser Asn Leu Val Ser Gly Asp Gly Gly Ala Ile Asp Ala
   145                 150                 155                 160
   Gln Ser Leu Met Val Asn Gly Ile Glu Lys Leu Cys Thr Phe Gln Glu
                   165                 170                 175
   Asn Val Ala Gln Ser Asp Gly Gly Ala Cys Gln Val Thr Lys Thr Phe
               180                 185                 190
   Ser Ala Val Gly Asn Lys Val Pro Leu Ser Phe Leu Gly Asn Val Ala
           195                 200                 205
   Gly Asn Lys Gly Gly Gly Val Ala Ala Val Lys Asp Gly Gln Gly Ala
       210                 215                 220
   Gly Gly Ala Thr Asp Leu Ser Val Asn Phe Ala Asn Asn Thr Ala Val
   225                 230                 235                 240
   Glu Phe Glu Gly Asn Ser Ala Arg Ile Gly Gly Gly Ile Tyr Ser Asp
                   245                 250                 255
   Gly Asn Ile Ser Phe Leu Gly Asn Ala Lys Thr Val Phe Leu Ser Asn
               260                 265                 270
   Val Ala Ser Pro Ile Tyr Val Asp Pro Ala Ala Ala Gly Gly Gln Pro
           275                 280                 285
   Pro Ala Asp Lys Asp Asn Tyr Gly Asp Gly Gly Ala Ile Phe Cys Lys
       290                 295                 300
   Asn Asp Thr Asn Ile Gly Glu Val Ser Phe Lys Asp Glu Gly Val Val
   305                 310                 315                 320
   Phe Phe Ser Lys Asn Ile Ala Ala Gly Lys Gly Gly Ala Ile Tyr Ala
                   325                 330                 335
   Lys Lys Leu Thr Ile Ser Asp Cys Gly Pro Val Gln Phe Leu Gly Asn
               340                 345                 350
   Val Ala Asn Asp Gly Gly Ala Ile Tyr Leu Val Asp Gln Gly Glu Leu
           355                 360                 365
   Ser Leu Ser Ala Asp Arg Gly Asp Ile Ile Phe Asp Gly Asn Leu Lys
       370                 375                 380
   Arg Met Ala Thr Gln Gly Ala Ala Thr Val His Asp Val Met Val Ala
   385                 390                 395                 400
   Ser Asn Ala Ile Ser Met Ala Thr Gly Gly Gln Ile Thr Thr Leu Arg
                   405                 410                 415
   Ala Lys Glu Gly Arg Arg Ile Leu Phe Asn Asp Pro Ile Glu Met Ala
               420                 425                 430
   Asn Gly Gln Pro Val Ile Gln Thr Leu Thr Val Asn Glu Gly Glu Gly
           435                 440                 445
   Tyr Thr Gly Asp Ile Val Phe Ala Lys Gly Asp Asn Val Leu Tyr Ser
       450                 455                 460
   Ser Ile Glu Leu Ser Gln Gly Arg Ile Ile Leu
   465                 470                 475
<210> 46
   <211> 550
   <212> PRT
   <213> Chlamydia muridarum
<400> 46
   Leu Lys Leu Pro Asn Leu Thr Phe Gly Gly Arg Glu Ile Glu Phe Ile
   1               5                   10                  15
   Val Thr Pro Pro Ser Ser Ile Ala Ala Gln Tyr Ile Thr Tyr Ala Asn
               20                  25                  30
   Val Ser Asn Tyr Arg Gly Asn Phe Thr Ile Ser Ser Cys Thr Gln Asp
           35                  40                  45
   Gln Trp Phe Ser Arg Gly Leu Ser Thr Thr Asn Ser Ser Gly Ala Phe
       50                  55                  60
   Val Glu Ser Met Thr Ser Phe Thr Ala Ile Asp Asn Ala Asp Leu Phe
   65                  70                  75                  80
   Phe Cys Asn Asn Tyr Cys Thr His Gln Gly Gly Gly Gly Ala Ile Asn
                   85                  90                  95
   Ala Thr Gly Leu Ile Ser Phe Lys Asn Asn Gln Asn Ile Leu Phe Tyr
               100                 105                 110
   Asn Asn Thr Thr Ile Gly Thr Gln Phe Thr Gly Val Ala Leu Arg Thr
           115                 120                 125
   Glu Arg Asn Arg Gly Gly Ala Leu Tyr Gly Ser Ser Ile Glu Leu Ile
       130                 135                 140
   Asn Asn His Ser Leu Asn Phe Ile Asn Asn Thr Ser Gly Asp Met Gly
   145                 150                 155                 160
   Gly Ala Val Ser Thr Ile Gln Asn Leu Val Ile Lys Asn Thr Ser Gly
                   165                 170                 175
   Ile Val Ala Phe Glu Asn Asn His Thr Thr Asp His Ile Pro Asn Thr
               180                 185                 190
   Phe Ala Thr Ile Leu Ala Arg Gly Gly Ala Val Gly Cys Gln Gly Ala
           195                 200                 205
   Cys Glu Ile Ser His Asn Thr Gly Pro Val Val Phe Asn Ser Asn Tyr
       210                 215                 220
   Gly Gly Tyr Gly Gly Ala Ile Ser Thr Gly Gly Gln Cys Ile Phe Arg
   225                 230                 235                 240
   Asp Asn Lys Asp Lys Leu Ile Phe Ile Asn Asn Ser Ala Leu Gly Trp
                   245                 250                 255
   His Asn Thr Ser Ala Gln Gly Asn Gly Ala Val Ile Ser Ala Gly Gly
               260                 265                 270
   Glu Phe Gly Leu Leu Asn Asn Lys Gly Pro Ile Tyr Phe Glu Asn Asn
           275                 280                 285
   Asn Ala Ser Tyr Ile Ala Gly Ala Ile Ser Cys Asn Asn Leu Asn Phe
       290                 295                 300
   Gln Glu Asn Gly Pro Ile Tyr Phe Leu Asn Asn Ser Ala Leu Tyr Gly
   305                 310                 315                 320
   Gly Ala Phe His Leu Phe Ala Ser Pro Ala Ala Asn Tyr Ile His Thr
                   325                 330                 335
   Gly Ser Gly Asp Ile Ile Phe Asn Asn Asn Thr Glu Leu Ser Thr Thr
                   340                 345                 350
   Gly Met Ser Ala Gly Leu Arg Lys Leu Phe Tyr Ile Pro Gly Thr Thr
           355                 360                 365
   Asn Asn Asn Pro Ile Thr Leu Ser Leu Gly Ala Lys Lys Asp Thr Arg
       370                 375                 380
   Ile Tyr Phe Tyr Asp Leu Phe Gln Trp Gly Gly Leu Lys Lys Ala Asn
   385                 390                 395                 400
   Thr Pro Pro Glu Asn Ser Pro His Thr Val Thr Ile Asn Pro Ser Asp
                   405                 410                 415
   Glu Phe Ser Gly Ala Val Val Phe Ser Tyr Lys Asn Ile Ser Ser Asp
               420                 425                 430
   Leu Gln Ala His Met Ile Ala Ser Lys Thr His Asn Gln Ile Lys Asp
           435                 440                 445
   Ser Pro Thr Thr Leu Lys Phe Gly Thr Met Ser Ile Glu Asn Gly Ala
       450                 455                 460
   Glu Phe Glu Phe Phe Asn Gly Pro Leu Thr Gln Glu Ser Thr Ser Leu
   465                 470                 475                 480
   Leu Ala Leu Gly Gln Asp Ser Ile Leu Thr Val Gly Lys Asp Ala Ser
                   485                 490                 495
   Leu Thr Ile Thr His Leu Gly Ile Ile Leu Pro Gly Leu Leu Asn Asp
               500                 505                 510
   Gln Gly Thr Thr Ala Pro Arg Ile Arg Val Asn Pro Gln Asp Met Thr
           515                 520                 525
   Gln Asn Thr Asn Ser Asn Gln Ala Pro Val Ser Thr Glu Asn Val Ala
       530                 535                 540
   Thr Gln Lys Ile Phe Phe
   545                 550
<210> 47
   <211> 367
   <212> PRT
   <213> Chlamydia Muridarum
<220>
   <221> misc_feature
   <222> (285)..(285)
   <223> Xaa can be any naturally occurring amino acid
<220>
   <221> misc_feature
   <222> (292)..(292)
   <223> Xaa can be any naturally occurring amino acid
<400> 47
   His Ala Leu Pro Val Gly Asn Pro Ala Glu Pro Ser Leu Met Ile Asp
   1               5                   10                  15
   Gly Ile Leu Trp Glu Gly Phe Gly Gly Asp Pro Cys Asp Pro Cys Thr
               20                  25                  30
   Thr Trp Cys Asp Ala Ile Ser Leu Arg Leu Gly Tyr Tyr Gly Asp Phe
           35                  40                  45
   Val Phe Asp Arg Val Leu Lys Thr Asp Val Asn Lys Gln Phe Glu Met
       50                  55                  60
   Gly Ala Ala Pro Thr Gly Asp Ala Asp Leu Thr Thr Ala Pro Thr Pro
   65                  70                  75                  80
   Ala Ser Arg Glu Asn Pro Ala Tyr Gly Lys His Met Gln Asp Ala Glu
                   85                  90                  95
   Met Phe Thr Asn Ala Ala Tyr Met Ala Leu Asn Ile Trp Asp Arg Phe
               100                 105                 110
   Asp Val Phe Cys Thr Leu Gly Ala Thr Ser Gly Tyr Leu Lys Gly Asn
           115                 120                 125
   Ser Ala Ala Phe Asn Leu Val Gly Leu Phe Gly Arg Asp Glu Thr Ala
       130                 135                 140
   Val Ala Ala Asp Asp Ile Pro Asn Val Ser Leu Ser Gln Ala Val Val
   145                 150                 155                 160
   Glu Leu Tyr Thr Asp Thr Ala Phe Ala Trp Ser Val Gly Ala Arg Ala
                   165                 170                 175
   Ala Leu Trp Glu Cys Gly Cys Ala Thr Leu Gly Ala Ser Phe Gln Tyr
               180                 185                 190
   Ala Gln Ser Lys Pro Lys Val Glu Glu Leu Asn Val Leu Cys Asn Ala
           195                 200                 205
   Ala Glu Phe Thr Ile Asn Lys Pro Lys Gly Tyr Val Gly Gln Glu Phe
       210                 215                 220
   Pro Leu Asn Ile Lys Ala Gly Thr Val Ser Ala Thr Asp Thr Lys Asp
   225                 230                 235                 240
   Ala Ser Ile Asp Tyr His Glu Trp Gln Ala Ser Leu Ala Leu Ser Tyr
                   245                 250                 255
   Arg Leu Asn Met Phe Thr Pro Tyr Ile Gly Val Lys Trp Ser Arg Ala
               260                 265                 270
   Ser Phe Asp Ala Asp Thr Ile Arg Ile Ala Gln Pro Xaa Leu Glu Thr
           275                 280                 285
   Ser Ile Leu Xaa Met Thr Thr Trp Asn Pro Thr Ile Ser Gly Ser Gly
       290                 295                 300
   Ile Asp Val Asp Thr Lys Ile Thr Asp Thr Leu Gln Ile Val Ser Leu
   305                 310                 315                 320
   Gln Leu Asn Lys Met Lys Ser Arg Lys Ser Cys Gly Leu Ala Ile Gly
                   325                 330                 335
   Thr Thr Ile Val Asp Ala Asp Lys Tyr Ala Val Thr Val Glu Thr Arg
               340                 345                 350
   Leu Ile Asp Glu Arg Ala Ala His Val Asn Ala Gln Phe Arg Phe
           355                 360                 365
<210> 48
   <211> 20
   <212> DNA
   <213> Artificial Sequence
<220>
   <223> CpG ODN
<400> 48
   tccatgacgt tcctgacgtt 20

## Description

### TECHNICAL FIELD

The present invention relates to the field of immunology, and immunostimulatory agents. More specifically, the present invention relates to compositions comprising *Chlamydia* antigens; the compositions may be useful for inducing an immune response to a *Chlamydia* spp.

### BACKGROUND

*C. trachomatis* includes three human biovars: trachoma (serovars A, B, Ba or C), urethritis (serovars D-K), and lymphogranuloma venereum (LGV, serovars L1, 2 and 3). C. *trachomatis* is a obligate intracellular pathogen (i.e. the bacterium lives within human cells) and can cause numerous disease states in both men and women. Both sexes can display urethritis, proctitis (rectal disease and bleeding), trachoma, and infertility. The bacterium can cause prostatitis and epididymitis in men. In women, cervicitis, pelvic inflammatory disease (PID), ectopic pregnancy, and acute or chronic pelvic pain are frequent complications. *C. trachomatis* is also an important neonatal pathogen, where it can lead to infections of the eye (trachoma) and pulmonary complications.

Worldwide *Chlamydia trachomatis* is responsible for over 92 million sexually transmitted infections and 85 million ocular infections annually. Public health programs have targeted *C. trachomatis* as a major problem because of the ability of the organism to cause long term sequelae such as infertility, ectopic pregnancy and blindness. In developed countries, public health measures to prevent and control *Chlamydia* appear to be failing as case rates continue to rise and in developing countries efforts to control *Chlamydia* are not feasible using current approaches.

Immunity to *Chlamydia* is known to depend on cell-mediated immune (CMI) responses, especially Th1 polarized cytokine responses (Brunham et al., 2005). Antibodies appear to play a secondary role. Experience has shown that developing vaccines for intracellular pathogens that require protective CMI is more difficult than for pathogens that simply require protective antibody. Part of the problem has been the identification of antigens that induce protective CMI responses because protective antigens need to be presented to T cells by MHC molecules and identifying MHC-bound microbial epitopes has been difficult. Immunity to *Chlamydia* can be induced using whole inactivated *C. trachomatis* elementary bodies, but the vaccine efficacy was both incomplete and short lived. Additionally, breakthrough *C. trachomatis* infection in primate models resulted in more severe disease with worse inflammation post-vaccination. Other vaccine efforts have focused on subunit vaccines that comprise individual *C. trachomatis* antigens. The *Chlamydia* major outer membrane protein (MOMP) has been evaluated as a vaccine candidate in primate models, yet the MOMP-based vaccine only conferred marginal protection (Kari et al. Fourth Meeting of the European Society for Chlamydia Research, Aarhus, Denmark, 1-4 July 2008).

Genomic-based approaches to identify candidate peptides, proteins, subunits or epitopes may provide an efficient method for identifying moieties with potential for use in a vaccine, particularly in the context of the well-studied mouse model. Li et al 2006 (Vaccine 24:2917-2927) used bioinformatic and PCR-based methods to produce cloned open reading frames (ORFs), which were in turn pool-inoculated into mice, with subsequent rounds of challenge and further screening to identify ORFs that demonstrated significant protection.

Making a vaccine for pathogens that require protective cell-mediated immunity (CMI) responses is more difficult than for pathogens which require protective antibody responses. Part of the problem has been the identification of individual antigens that induce protective CMI responses. Studies in animal models and during human infection have established that *Chlamydia-*specific CD4+ T cells producing gamma interferon (IFN-gamma) are critically involved in the clearance of a *Chlamydia* infection (Su et al. 1995 Infect Immun 63:3302-3308; Wang et al. 1999 Eur J Immunol 29:3782-3792)*.* Design of an effective vaccine for a chlamydial infection may require the selection of antigens that effectively stimulates CD4+ Th1 cells.

Patents and patent applications disclosing nucleic acid or polypeptide compositions comprising full or partial MOMP sequences are described in, for example, US 6030799, US 6696421, US6676949, US 6464979, US 6653461 and US Patent Publication 2008/0102112.

Other *Chlamydia* sequences (nucleic acid and polypeptide) are described in, for example, US 6642023, US 6887843 and US 7459524; and US Patent Publications 2005/0232941, 2009/0022755, 2005/0035296, 2006/0286128.
Karunakaran et al (2008) Journal of Immunology v180, p 2459-2465, describes synthetic peptides derived from *Chlamydia* proteins and their use *in vivo* to demonstrate resistance to Chlamydia infection.

### SUMMARY OF THE INVENTION

The present invention relates to the field of immunology, and immunostimulatory agents. More specifically, the present invention relates to compositions comprising *Chlamydia* antigens; the compositions may be useful for inducing an immune response to a *Chlamydia* spp.

In accordance with one aspect of the invention, there is provided a composition comprising PmpG, Pmpf PmpF, and major outer membrane protein 1 (MOMP) polypeptide in combination with an adjuvant, wherein the PmpG polypeptide comprises or consists of an amino acid sequence set forth in SEQ ID NO: 13, SEQ ID NO: 42, or SEQ ID NO: 45; the PmpF polypeptide comprises an amino acid sequence set forth in SEQ ID NO: 16, SEQ ID NO: 43, or SEQ ID NO: 46; and the MOMP polypeptide comprises an amino acid sequence set forth in SEQ ID NO: 44 or SEQ ID NO: 47.

.

The adjuvant may be dimethyldioctadecylammonium bromide and trehalose 6,6'-dibehenate (DDA/TDB) or AbISCO. The *Chlamydia* species may be C. *trachomatis* or C. *muridarum.*

The immune response may be a cellular immune response.

The polypeptides may be *Chlamydia trachomatis* polypeptides, or *Chlamydia muridarum* polypeptides.

The composition may comprise the PmpG polypeptide comprising an amino acid sequence set forth in SEQ ID NO: 42; the PmpF polypeptide comprising an amino acid sequence set forth in SEQ ID NO: 43; the MOMP polypeptide comprising an amino acid sequence set forth in SEQ ID NO: 44; and the adjuvant dimethyldioctadecylammonium bromide and trehalose 6,6'-dibehenate (DDA/TDB).

In another aspect of the invention, a composition of the invention may be used for the treatment or prevention of a *Chlamydia* infection in a subject. The *Chlamydia* infection may be in a lung or a genital tract. The *Chlamydia* infection may be associated with *C. trachomatis.* The subject may be human. The composition may be for intranasal administration or for injection.

The examples provided herein demonstrate that a *Chlamydia* vaccine based on recombinant C.*muridarum* proteins (PmpG-1, PmpE/F-2 and MOMP) or fragments thereof and formulated with a liposome adjuvant DDA/TDB is protective against vaginal challenge with *C. muridarum.* This protection correlates with strong IFN-γ, TNF-α and IL-17 responses characterized by the high frequency of IFN-γ/ TNF-α double positive CD4+ T cells and IFN-γ/IL-17 double positive CD4+ T cells.

This summary of the invention does not necessarily describe all features of the invention. Other aspects, features and advantages of the present invention will become apparent to those of ordinary skill in the art upon review of the following description of specific embodiments of the invention.

### BRIEF DESCRIPTION OF THE DRAWINGS

These and other features of the invention will become more apparent from the following description in which reference is made to the appended drawings wherein:

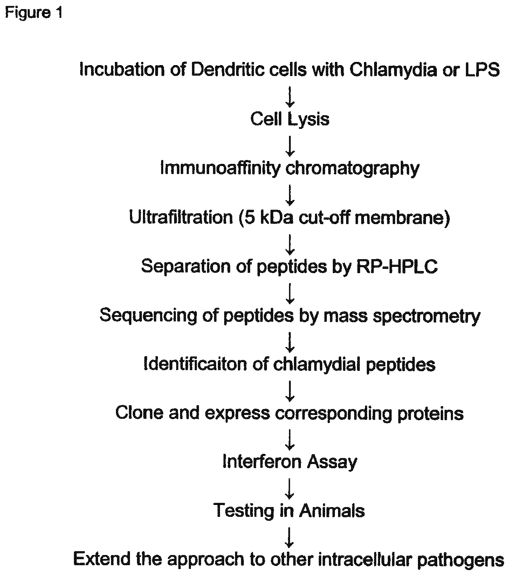

**Figure 1** shows an exemplary schematic of vaccine development.

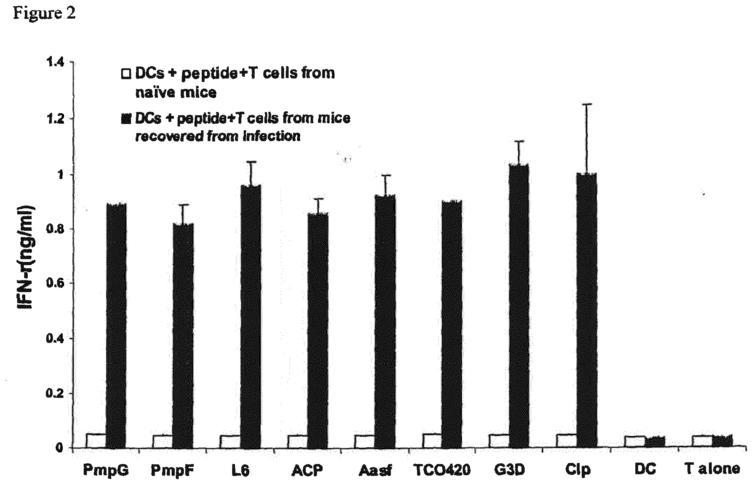

**Figure 2** shows the results of an ELISA assay to quantify interferon (IFN)-gamma production by CD4 T cells following exposure to dendritic cells that have been pulsed with a *C. muridarum* peptide; PmpG = Polymorphic membrane protein G (SEQ ID NO:13 - ASPIYVDPAAAGGQPPA), PmpF = Polymorphic membrane protein F (SEQ ID NO:16 - AFHLFASPAANYIHTG), L6 = Ribosomal protein L/Rplf (SEQ ID NO:10 - GNEVFVSPAAHIIDRPG), ACP = 3-oxoacyl-(acyl carrier protein) reductase (SEQ ID NO: 11 - SPGQTNYAAAKAGIIGFS), Aasf =Anti-anti-sigma factor (SEQ ID NO: 12 - KLDGVSSPAVQESISE), TC0420 = Hypothetical protein (SEQ ID NO: 14 - DLNVTGPKIQTDVD), G3D = Glyceraldehyde 3-phosphate dehydrogenase (SEQ ID NO:17 - MTTVHAATATQSVVD), Clp = ATP-dependent Clp protease proteolytic subunit (SEQ ID NO: 15 - IGQEITEPLANTVIA). As controls, T cells were also cultured alone (T alone) or with dendritic cells without the addition of any peptide antigen (DC). Black bars indicate that T cells were isolated from mice that had recovered from *Chlamydia* infection. White bars indicate that the T cells were isolated from naive mice.

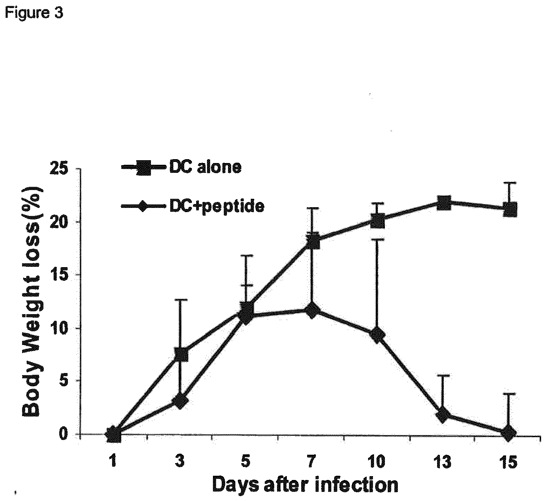

**Figure 3** shows the resistance to *Chlamydia muridarum* infection in mice following the adoptive transfer of dendritic cells that have been pulsed with *Chlamydia* peptides. LPS-treated dendritic cells were either left untreated (DC alone, black squares) or were pulsed with the eight *Chlamydia muridarum* MHC class II peptides (SEQ ID NOs: 10-17) (DC+ peptide, black diamonds). The dendritic cells were adoptively transferred to naive C57BL/6 mice that were subsequently challenged intranasally with 2000 Inclusion Forming Units (IFU) of *C. muridarum.* The results depict the percentage body weight loss of the mice following infection.

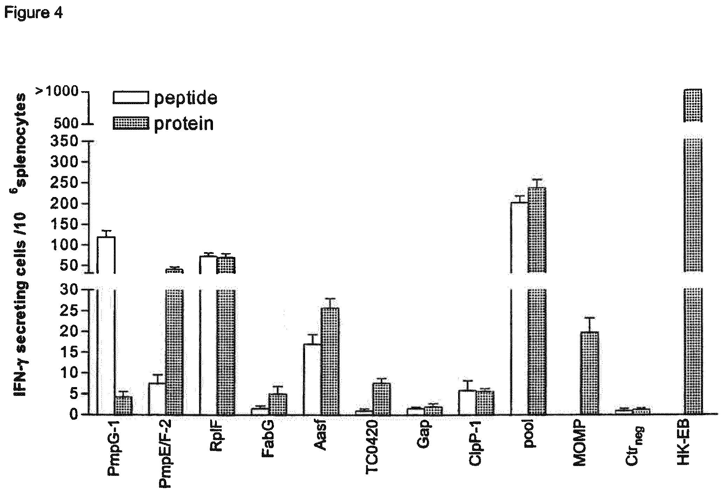

**Figure 4** shows the results of an ELISA assay to quantify interferon (IFN)-gamma production by splenocytes recovered from mice infected with *C. muridarum.* Mice were infected with intranasally with 1000 IFU live *C. muridarum.* One month later, the splenocytes from recovered mice were harvested and stimulated with *in vitro* for 20 h with 2 µg/ml individual peptide corresponding to SEQ ID NO: 10-17 (white bars) or a pool of peptides corresponding to SEQ ID NO: 10-17 (pool, white bar), 1 µg/ml individual polypeptides corresponding to Ribosomal protein L6 (RplF), 3_oxoacyl_(acyl carrier protein) reductase (FabG), Anti anti sigma factor (Aasf), Polymorphic membrane protein G (PmpG-1), Hypothetical protein TC0420, ATP_dependent Clp protease_proteolytic subunit (Clp), Polymorphic membrane protein F (PmpE/F) (hatched bars) or a pool of proteins (RplF, FabG, Aasf, PmpG-1, TC0420, Clp and PmpE/F).. One irrelevant OVA peptide (Ctr_{neg} white bar) and GST protein (Ctr_{neg} hatched bar) were used as peptide and protein negative controls, respectively. Heat killed EB (HK-EB) was used as a positive control. MOMP protein stimulation was also set up as a reference. The results represent the average of duplicate wells and are expressed as the means ± SEM of *Chlamydia muridarum* antigen-induced IFN-γ secreting cells per 10⁶ splenocytes for groups of six mice.

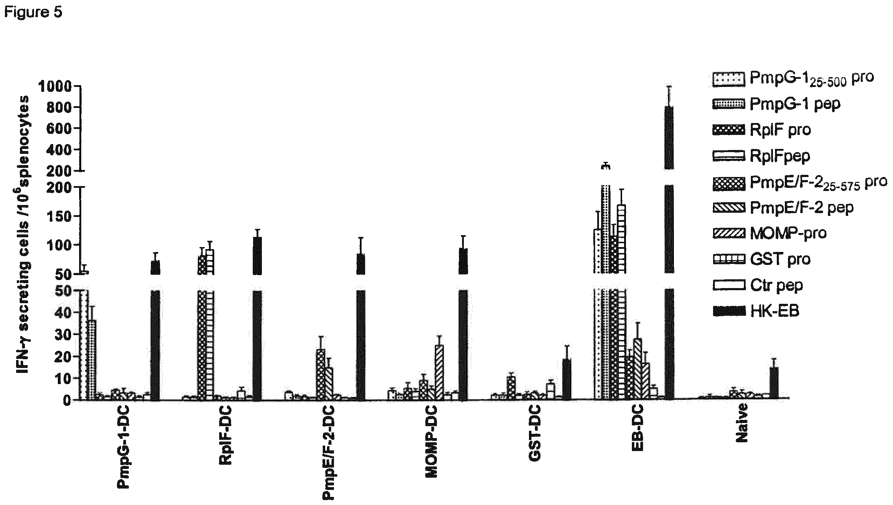

**Figure 5** shows the results of an ELISA assay to quantify interferon (IFN)-gamma production by splenocytes recovered from mice following the adoptive transfer of DCs transfected with *Chlamydia muridarum* polypeptides. Mice were vaccinated three times with DCs transfected with *Chlamydia muridarum* polypeptide PmpG-1₂₅₋₅₀₀ (PmpG-1-DC), RplF (RplF-DC), PmpE/F-2₂₅₋₅₇₅ (PmpE/F-2-DC) or MOMP (MOMP-DC) and matured overnight with LPS. DCs pulsed with live *C. muridarum* (EB-DC) or GST protein (GST-DC) was used as positive and negative controls, respectively. Two weeks after the last immunization, the splenocytes of each group were harvested for IFN-gamma ELISPOT assay. The results are expressed as the means ± SEM of *Chlamydia* antigen-induced IFN-gamma secreting cells per 10⁶ splenocytes for groups of six mice.

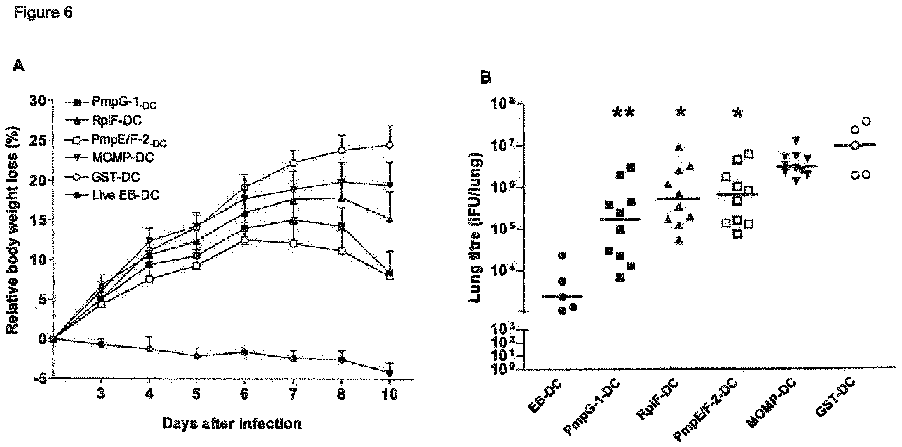

**Figure 6** shows the resistance to *Chlamydia* pulmonary infection in mice following the adoptive transfer of DCs transfected with *Chlamydia* proteins. Mice were adoptively transferred with DCs that were transfected with either the PmpG-1₂₅₋₅₀₀ (PmpG-1-DC), RplF (RplF-DC), PmpE/F-2₂₅₋₅₇₅ (PmpE/F-2-DC), MOMP protein (MOMP-DC) or the GST protein (GST-DC). DCs pulsed with live *C. muridarum* (EB-DC) was used as a positive control. Two weeks after the last immunization, mice were challenged intranasally with 2000 IFU live *C. muridarum.* (A) Weight loss was monitored each or every two days after challenge. (B) Ten days after intranasal challenge, the lungs were collected and bacterial titers were measured on HeLa 229 cells. *, *p* < 0.05; **, *p* < 0.01 as compared to the GST-DC group.

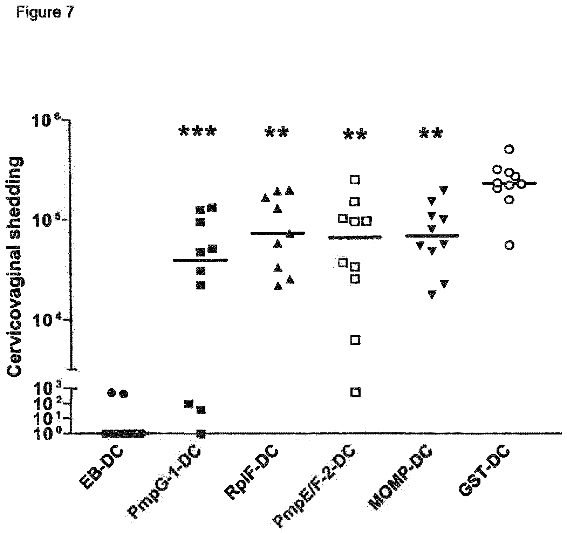

**Figure 7** shows the resistance to *Chlamydia* genital tract infection following adoptive transfer of DCs transfected with *Chlamydia* proteins. Mice were adoptively transferred with DCs that were transfected with either the PmpG-1₂₅₋₅₀₀ protein (PmpG-1-DC), the RplF protein (RplF-DC), the PmpE/F-2₂₅₋₅₇₅ protein (PmpE/F-2-DC), the MOMP protein (MOMP-DC) or the GST protein (GST-DC). DCs pulsed with live *C. muridarum* (EB-DC) was used as a positive control. One week after the final immunization, the mice from each group were injected with Depo-Provera. One week after Depo-Provera treatment, the mice were infected intravaginally with 1500 IFU live *C. muridarum.* Cervicovaginal washes were taken at day 6 after infection and bacterial titer were measured on HeLa 229 cells. *, *p* < 0.05; **, *p* < 0.01; ***, *p* < 0.001 as compared to the GST-DC group.

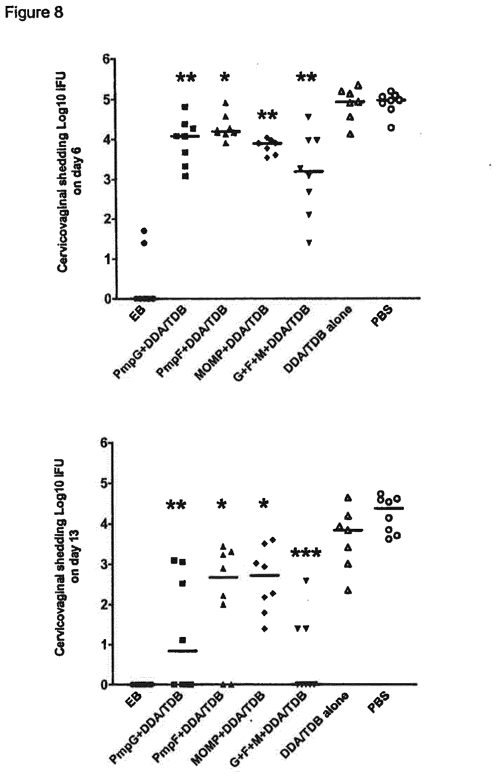

**Figure 8** Resistance to *Chlamydia* genital tract infection following subcutaneous vaccination with PmpG, PmpF, or MOMP protein or their combination formulated with adjuvant DDA/TDB. C57BL/6 mice were vaccinated three times with a 2-week interval with PBS, DDA/TDB alone as negative controls and live *Chlamydia* EB as positive control. G+F+M+DDA/TDB - PmpG, PmpF and MOMP combined with DDA/TDB. One week after the final immunization, the mice from each group were injected with Depo-Provera. One week after Depo-Provera treatment, the mice were infected intravaginally with 1500 IFU live *C. muridarum.* Cervicovaginal washes were taken at day 6 and day 13 after infection and bacterial titer were measured on HeLa 229 cells. The data shown above is at day 13. *, *p <* 0.05; **, *p* <0.01; ***, *p* < 0.001 *vs*. adjuvant alone group.

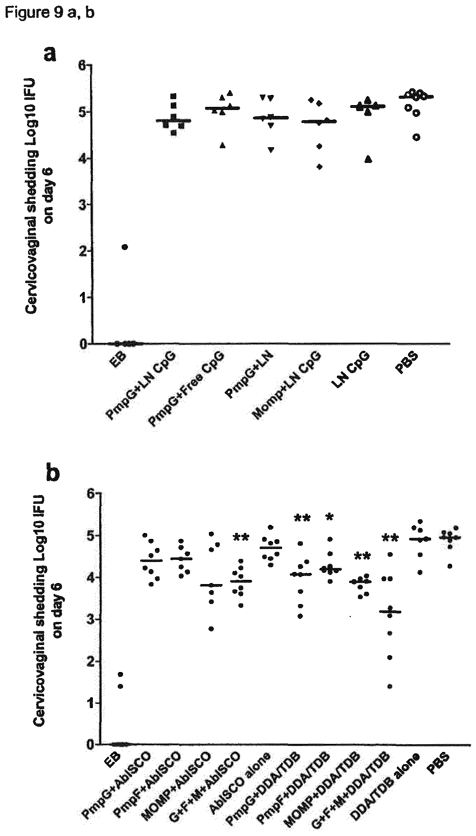

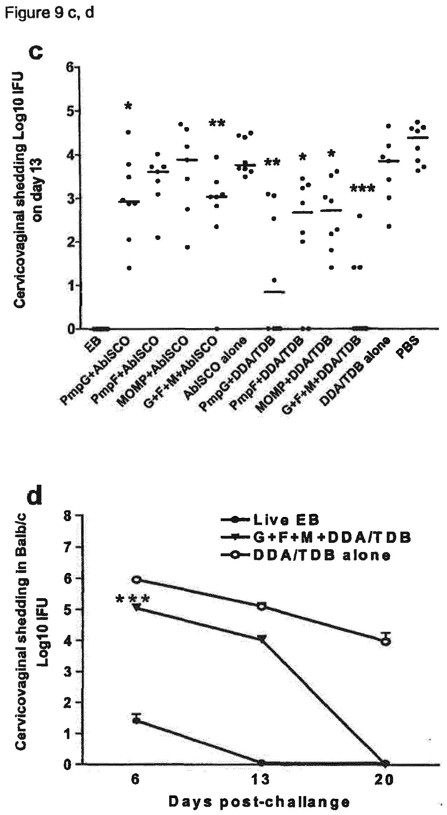

**Figure 9** shows vaccine-elicited protection against *Chlamydia* genital tract infection. Mice were intravaginally challenged with 1500 IFU live *C. muridarum* after immunization with a variety of vaccine formulation. Cervicovaginal washes were taken at selected dates after infection and bacterial titers were measured on HeLa 229 cells. *, *p* < 0.05; **, *p* < 0.01; ***, *p <* 0.001 *vs.* adjuvant alone group. (a) Failure to induce protection after vaccination of PmpG-1 or MOMP protein formulated with CpG ODN. (b) and (c) Resistance to *Chlamydia* infection in C57 mice immunized with PmpG-1, PmpE/F-2, MOMP protein or their combination formulated with adjuvant AbISCO-100 or DDA/TDB. Cervicovaginal washes were taken at day 6 (b) and day 13 (c) after infection. (d) Resistance to *Chlamydia* infection in BALB/c mice (n=8) immunized with the combination of PmpG-1, PmpE/F-2, MOMP protein formulated with adjuvant DDA/TDB.

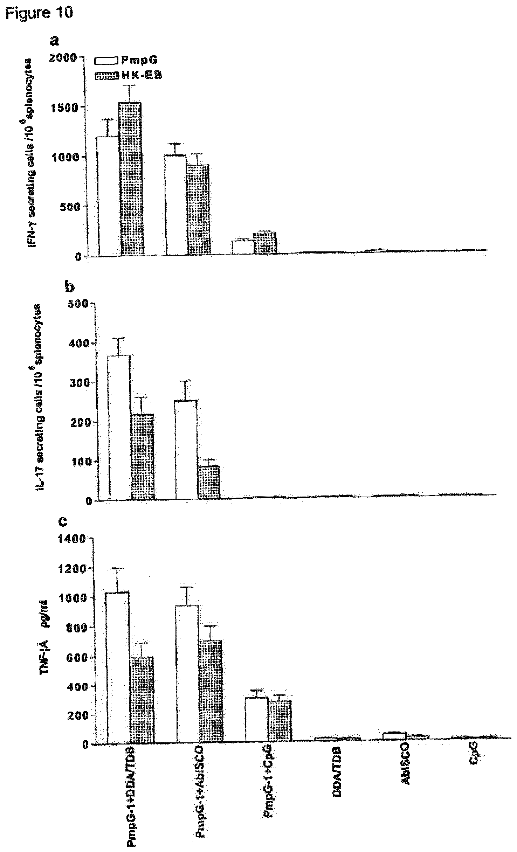

**Figure 10** shows *Chlamydia* antigen-specific cytokine response after immunization with PmpG-1 protein formulated with DDA/TDB, AbISCO or CpG adjuvants. Two weeks after the final immunization, mouse splenocytes from different vaccine groups were harvested and stimulated with 1 µg/ml PmpG-1 protein or 5×10₅ inclusion-forming units (IFU)/ml HK-EB. DDA/TDB alone, AbISCO alone or CpG alone adjuvants was set up as negative controls. The results represent the average of duplicate wells and are expressed as the means ± SEM for groups of six mice. (a) IFN-γ responses to PmpG-1 and HK-EB detected by ELISPOT assay. (b) IL-17 responses to PmpG-1 and HK-EB detected by ELISPOT assay. (c) TNF-α response to PmpG-1 and HK-EB detected by ELISA.

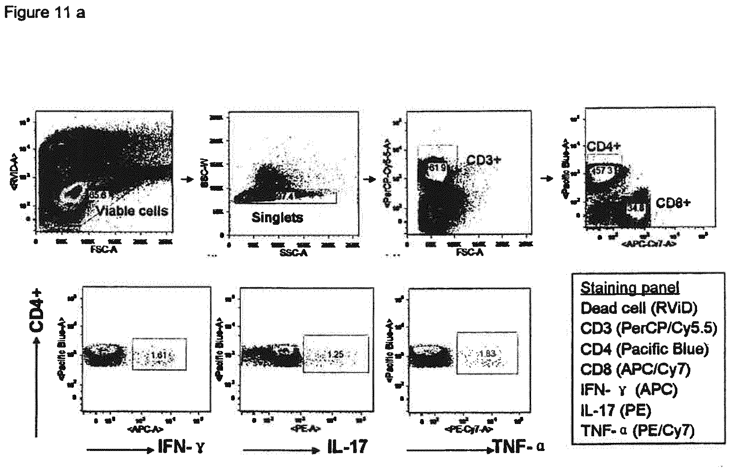

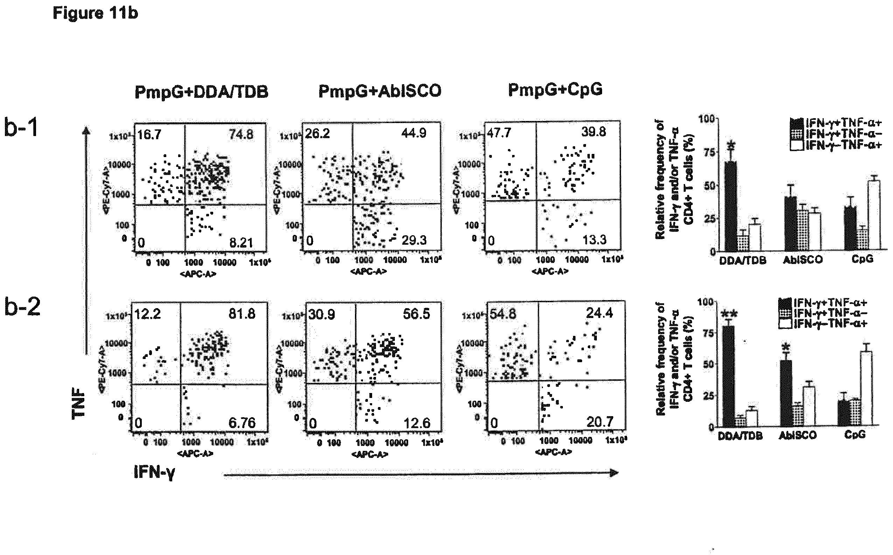

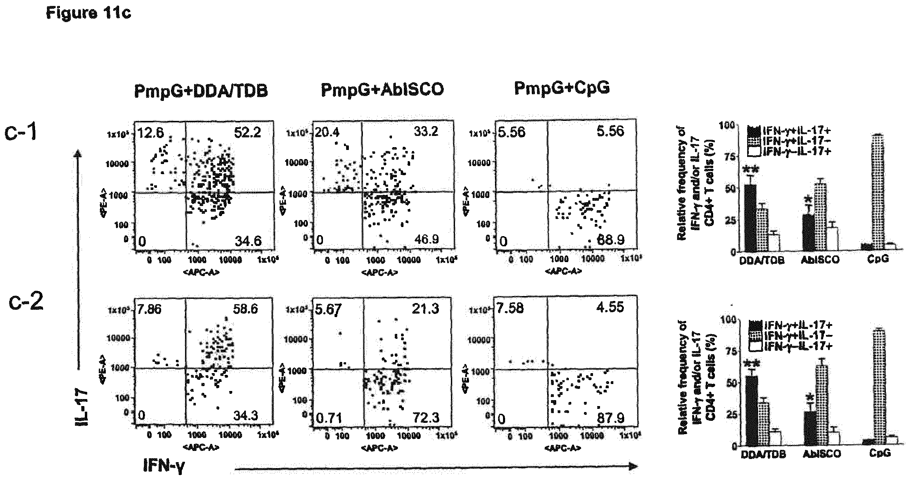

**Figure 11** shows functional characterization of distinct populations of *Chlamydia* antigen-specific cytokine responses after immunization. Splenocytes from different vaccine groups were analyzed by multiparameter flow cytometry. Three or four mice were in each group. Shown is the representative of two experiments. (a) The staining panel and gating strategy used to identify IFN-γ, TNF-α and IL-17 producing CD4+ T cells in the splenocytes from a representative mouse immunized with PmpG+DDA/TDB. (b) Comparison of the quality of CD4+ IFN-γ/ TNF-α responses to PmpG-1 protein (b-1) or HK-EB (b-2) in different vaccine groups. (c) Comparison of the quality of CD4+ IFN-γ/ IL-17 responses to PmpG-1 protein (c-1) or HK-EB (c-2) in different vaccine groups.

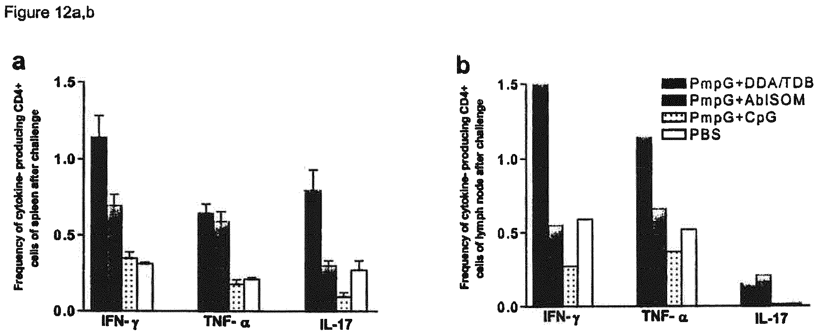

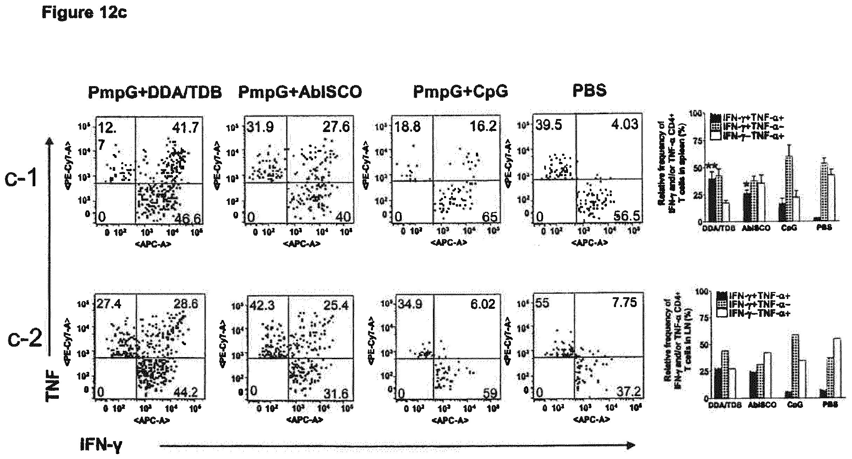

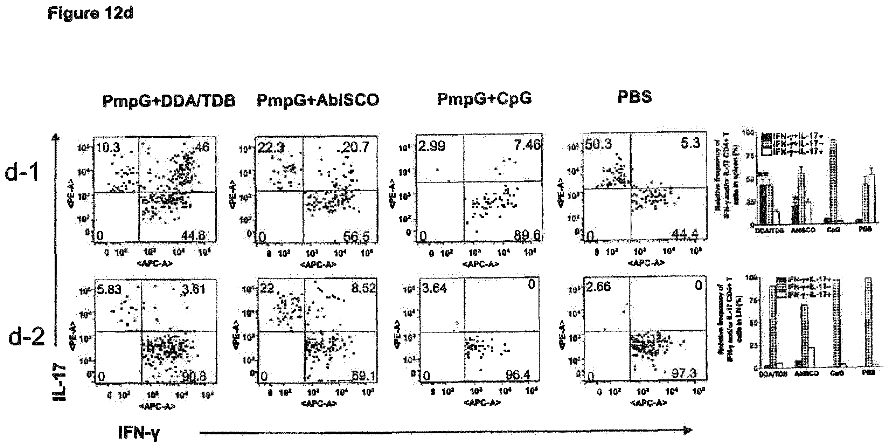

**Figure 12** shows the magnitude and quality of *Chlamydia* antigen-specific cytokine responses in spleen and draining lymph node after challenge. Splenocytes and draining lymph node (iliac lymph node) from different vaccine groups were analyzed by multiparameter flow cytometry as described in Methods and Materials. Four mice were studied in each group. (a) The total frequency of IFN-γ, TNF-α or IL-17 producing CD4+ T cell in spleens. (b) The total frequency of IFN-γ, TNF-α or IL-17 producing CD4+ T cell in iliac lymph node. (c) Comparison of the quality of CD4+ IFN-γ/ TNF-α responses to PmpG-1 protein in spleen (c-1) and in iliac lymph node (c-2) from different vaccine groups. (d) Comparison of the quality of CD4+ IFN-γ/ IL-17 responses to PmpG-1 protein in spleen (d-1) and in iliac lymph node (d-2) from different vaccine groups.

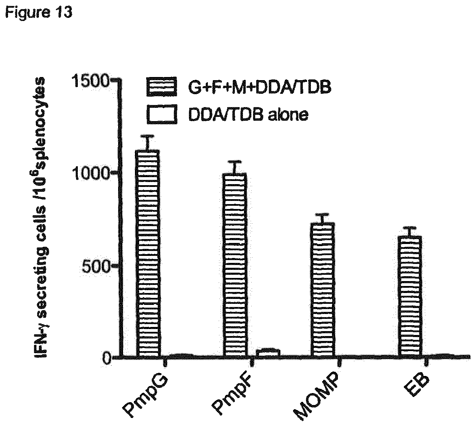

**Figure 13** shows Human *Chlamydia trachomatis* antigen-specific IFN-gamma response in mice after immunization with a cocktail of C. *trachomatis* serovar D proteins PmpG (SEQ ID NO: 42), PmpF (SEQ ID NO: 43) and MOMP (SEQ ID NO: 44) formulated with DDA/TDB adjuvant detected by ELISPOT assay. C57 BL/6 mice were immunized three times subcutaneously in the base of tail at 2-week intervals. Two weeks after the final immunization, splenocytes were harvested and stimulated with 1 microgram/ml *C. trachomatis* serovar D protein PmpG, PmpF, MOMP or 5X10⁵ inclusion-forming units (IFU) /ml heat-killed EB respectively. DDA/TDB alone adjuvant was set up as a negative control. The results represent the average of duplicate wells and are expressed as means ± SEM for groups of six mice.

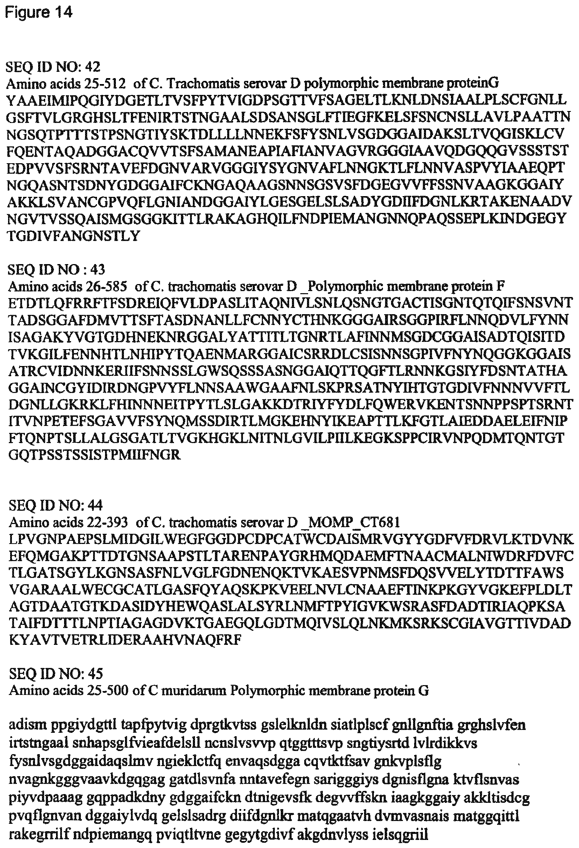

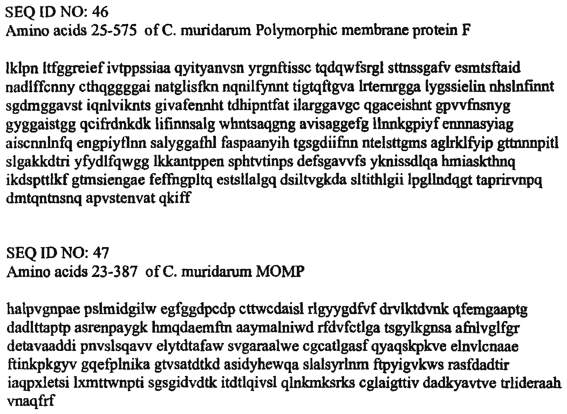

**Figure 14** shows polypeptide sequences according to SEQ ID NO: 42-47.

### DETAILED DESCRIPTION

The present invention relates to immunology, and immunostimulatory agents. More specifically, the present invention relates to compositions comprising *Chlamydia* antigens; the compositions may be useful for inducing an immune response to a *Chlamydia* spp.

In the description that follows, a number of terms are used extensively, the following definitions are provided to facilitate understanding of various aspects of the invention. Use of examples in the specification, including examples of terms, is for illustrative purposes only and is not intended to limit the scope and meaning of the embodiments of the invention herein.

Any terms not directly defined herein shall be understood to have the meanings commonly associated with them as understood within the art of the invention. Certain terms are discussed below, or elsewhere in the specification, to provide additional guidance to the practitioner in describing the devices, methods and the like of embodiments of the invention, and how to make or use them. It will be appreciated that the same thing may be said in more than one way. Consequently, alternative language and synonyms may be used for any one or more of the terms discussed herein. No significance is to be placed upon whether or not a term is elaborated or discussed herein. Some synonyms or substitutable methods, materials and the like are provided. Recital of one or a few synonyms or equivalents does not exclude use of other synonyms or equivalents, unless it is explicitly stated. Use of examples in the specification, including examples of terms, is for illustrative purposes only and does not limit the scope and meaning of the embodiments of the invention herein.

The present invention relates to compositions for inducing an immune response to a *Chlamydia* species in a subject. The compositions comprise one or more than one polypeptides of *Chlamydia trachomatis* or *Chlamydia muridarum,* or C. *trachomatis* and C. *muridarum.*

*Chlamydia* research is aided by a recognized murine model of infection that has been standardized (Brunham et al 2005. Nature Reviews Immunology 5:149-161; Taylor-Robinson and Tuffrey 1987. Infection and Immunity 24(2) 169-173; Pal et al 1998. Journal of Medical Microbiology 47(7) 599-605).

*C. muridarum* and *C. trachomatis* are highly orthologous pathogenic microbes having co-evolved with their host species. Of the approximately 1,000 genes that each organism has, all but six are shared between the two genomes. Differences in gene content between the two genomes are principally located at the replication termination region or plasticity zone. Within this region are found species specific genes that relate to host specific immune evasion mechanisms. Genes are found in *C. trachomatis* which encode tryptophan synthetase thereby allowing *C. trachomatis* to partially escape IFN-γ induced IDO-mediated tryptophan depletion in human cells. Mouse epithelial cells lack IDO and instead IFN-γ disrupts vesicular trafficking of sphingomyelin to the inclusion. *C. muridarum* in its genome has several genes which encode an intracellular toxin that disrupts vesicular trafficking thereby enabling partial escape from IFN-γ inhibition in murine cells.

Extraordinary gene conservation is shared between two microbial genomes. Without wishing to be bound by theory, this high degree of genome similarity may be due to the fact that as an intracellular pathogen *Chlamydiae* rarely undergoes lateral gene transfer events. Most genome differences result from accumulated point mutations and gene duplication. For genes shared between the two *Chlamydia* species, encoded proteins differ in sequence on average about 20% reflecting the extended period of time the two species have been evolutionarily separated.

In part, because the two genomes are so highly orthologous, immune responses to infection are very similar between the two host species. Because *C. muridarum,* like human strains, is indifferent to innate interferon gamma defenses in its natural host, clearance in the murine model is dependent on adaptive immunity, and therefore *C. muridarum* can serve as a robust animal model for studying cellular immunity and vaccine development. In both mice and humans CD4 T cells are particularly important to clearance of infection. Antibodies to surface macromolecules may synergise with CD4 Th1 mediated immunity in preventing reinfection. CD4 Th2 and CD4 Th17 responses in the absence of Th1 responses correlate with tissue pathology and persistent infection.

Thus, the mouse model of *C. muridarum* infection may be useful to elucidate the immunobiology of T cell responses and guide the design of a molecular vaccine to prevent human *C. trachomatis* infection.

Various *Chlamydia* spp have had the genome sequence determined, and the sequences of the expressed polypeptides determined. The genome sequence of *C. trachomatis* is described in Stephens, R.S. et al., 1998 (Genome sequence of an obligate intracellular pathogen of humans: Chlamydia trachomatis. Science 282 (5389): 754-759). Examples of expressed polypeptides of *C. trachomatis* that may be included in compositions according to various embodiments described herein include amino acid permease (gi:3328837), Ribosomal protein L6 (RplF, gi:3328951), 3-oxoacyl - (acyl carrier protein) reductase (FabG, gi:15604958), Anti anti sigma factor (Aasf, gi:15605151), Polymorphic membrane protein G (PmpG, gi:3329346), Hypothetical protein (TC0420, gi:15604862), ATP dependent Clp protease (Clp1, gi:15605439), Polymorphic membrane protein F (PmpF, gi:3329345), Glyceraldehyde 3-phosphate dehydrogenase (Gap, gi:15605234) and major outer membrane protein 1 (MOMP) (gi:3329133), or fragments or portions thereof. Examples of fragments or portions of the above-referenced polypeptides include amino acids 25 - 512 of PmpG (PmpG₂₅₋₅₁₂) (SEQ ID NO: 42), amino acids 26-585 of PmpF (PmpF₂₆₋₅₈₅) (SEQ ID NO: 43), and amino acids 22-393 of MOMP (SEQ ID NO: 44).

The genome sequence of *C*. *muridarum* is described in Read,T., et al., 2000 (Genome sequences of Chlamydia trachomatis MoPn and Chlamydia pneumoniae AR39 Nucleic Acids Res. 28 (6): 1397-1406). Examples of expressed polypeptides of *C*. *muridarum* that may be included in compositions according to various embodiments described herein, or employed in various experimental examples described herein include amino acid permease (gi: 15835268), Ribosomal protein L6 (RplF, gi: 15835415), 3_oxoacyl_(acyl carrier protein) reductase (FabG, gi:15835126), Anti anti sigma factor (Aasf, gi:15835322), Polymorphic membrane protein G (PmpG or PmpG-1, gi:15834883), Hypothetical protein TC0420(gi:15835038), ATP_dependent Clp protease_proteolytic subunit (Clp, gi:15834704), Polymorphic membrane protein F (PmpF or PmpE/F, gi:15834882), Glyceraldehyde 3_phosphate dehydrogenase (Gap, gi:15835406) and major outer membrane protein 1 (MOMP, gi7190091), or fragments or portions thereof. Examples of fragments or portions of the above-referenced polypeptides include amino acids 25 - 500 of PmpG-1 (PmpG-1₂₅₋₅₀₀) (SEQ ID NO: 45), amino acids 25-575 of PmpE/F-2 (PmpE/F-2₂₅₋₅₇₅) (SEQ ID NO: 46), and amino acids 23 -387end of MOMP (SEQ ID NO: 47).

The nucleotide and amino acid sequences of MOMP are also described in, for example, US 6838085 and US 6344302.

In some examples, the one, or more than one, polypeptide may be selected from the group consisting of SEQ ID NO: 10, SEQ ID NO: 13, SEQ ID NO: 16, SEQ ID NO:17, SEQ ID NO: 20, SEQ ID NO: 21, SEQ ID NO: 22, SEQ ID NO: 23, SEQ ID NO: 27, SEQ ID NO: 28, SEQ ID NO: 31, SEQ ID NO: 42, SEQ ID NO: 43, SEQ ID NO: 44, SEQ ID NO: 45, SEQ ID NO: 46, SEQ ID NO: 47 and RplF, and an excipient.

The one or more than one polypeptides may be from *Chlamydia trachomatis,* or C. *muridarum.*

A fragment or portion of a protein, fusion protein or polypeptide includes a peptide or polypeptide comprising a subset of the amino acid complement of a particular protein or polypeptide. The fragment may, for example, comprise an antigenic region or a region comprising a functional domain of the protein or polypeptide. The fragment may also comprise a region or domain common to proteins of the same general family, or the fragment may include sufficient amino acid sequence to specifically identify the full-length protein from which it is derived. In some embodiments, the fragment may specifically exclude signal peptides for translocation to organelles or membranes of the cell. In some embodiments, the fragment may comprise a region or domain found on the external surface of the cell (e.g. an outer membrane protein or portion thereof) when the polypeptide is expressed in the organism or cell.

For example, a fragment or portion may comprise from about 20% to about 100%, of the length of the full length of the protein, or any amount therebetween. For example, from about 20% to about 100%, 30% to about 100%, 40% to about 100%, 50% to about 100%, 60% to about 100%, from about 70% to about 100%, from about 80% to about 100%, from about 90% to about 100%, from about 95% to about 100%, of the length of the full length of the protein, or any amount therebetween. Alternately, a fragment or portion may be from about 50 to about 500 amino acids, or any amount therebetween. For example, a fragment may be from 50 to about 500 amino acids, or any amount therebetween, from about 75 to about 500 amino acids or any amount therebetween, from about 100 to about 500 amino acids or any amount therebetween, from about 125 to about 500 amino acids or any amount therebetween, from about 150 to about 500 amino acids, or any amount therebetween, from about 200 to about 500 amino acids, or any amount therebetween, from about 250 to about 500 amino acids, or any amount therebetween, from about 300 to about 500 or any amount therebetween, from about 350 to about 500 amino acids, or any amount therebetween, from about 400 to about 500 or any amount therebetween, from about 450 to about 500 or any amount therebetween, depending upon the HA, and provided that the fragment can form a VLP when expressed. For example, about 5, 10, 20, 30, 40 or 50 amino acids, or any amount therebetween may be removed from the C terminus, the N terminus or both the N and C terminus.

Numbering of amino acids in any given sequence are relative to the particular sequence, however one of skill can readily determine the 'equivalency' of a particular amino acid in a sequence based on structure and/or sequence. For example, if 6 N terminal amino acids were removed when constructing a clone for crystallography, this would change the specific numerical identity of the amino acid (e.g. relative to the full length of the protein), but would not alter the relative position of the amino acid in the structure.

The composition of the invention may be used in a method of inducing or eliciting an immune response against C. *trachomatis* or C. *muridarum* in a subject, comprising administration of a composition comprising one or more C. *trachomatis,* or C. *muridarum,* or C. *trachomatis* and C *muridarum* polypeptides, and an excipient. The composition may further comprise an adjuvant,a delivery agent, or an adjuvant and a delivery agent.

Antigen presenting cells (APCs) such as dendritic cells (DCs) take up polypeptides and present epitopes of such polypeptides within the context of the DC MHC I and II complexes to other immune cells including CD4+ and CD8+ cells. An 'MHC complex' or 'MHC receptor' is a cell-surface receptor encoded by the major histocompatibility complex of a subject, with a role in antigen presentation for the immune system. MHC proteins may be found on several cell types, including antigen presenting cells (APCs) such as macrophages or dendritic cells (DCs), or other cells found in a mammal. Epitopes associated with MHC Class I may range from about 8-11 amino acids in length, while epitopes associated MHC Class II may be longer, ranging from about 9-25 amino acids in length.

The term "epitope" refers to an arrangement of amino acids in a protein or modifications thereon (for example glycosylation). The amino acids may be arranged in a linear fashion, such as a primary sequence of a protein, or may be a secondary or tertiary arrangement of amino acids in close proximity once a protein is partially or fully configured. Epitopes may be specifically bound by an antibody, antibody fragment, peptide, peptidomimetic or the like, or may be specifically bound by a ligand or held within an MHC I or MHC II complex. An epitope may have a range of sizes - for example a linear epitope may be as small as two amino acids, or may be larger, from about 3 amino acids to about 20 amino acids. In some embodiments, an epitope may be from about 5 amino acids to about 10 or about 15 amino acids in length. An epitope of secondary or tertiary arrangements of amino acids may encompass as few as two amino acids, or may be larger, from about 3 amino acids to about 20 amino acids. In some embodiments, a secondary or tertiary epitope may be from about 5 amino acids to about 10 or about 15 amino acids in proximity to some or others within the epitope.

An "immune response" generally refers to a response of the adaptive immune system. The adaptive immune system generally comprises a humoral response, and a cell-mediated response. The humoral response is the aspect of immunity that is mediated by secreted antibodies, produced in the cells of the B lymphocyte lineage (B cell). Secreted antibodies bind to antigens on the surfaces of invading microbes (such as viruses or bacteria), which flags them for destruction. Humoral immunity is used generally to refer to antibody production and the processes that accompany it, as well as the effector functions of antibodies, including Th2 cell activation and cytokine production, memory cell generation, opsonin promotion of phagocytosis, pathogen elimination and the like. The terms "modulate" or "modulation" or the like refer to an increase or decrease in a particular response or parameter, as determined by any of several assays generally known or used, some of which are exemplified herein. The cellular processes involved in stimulation of B-cells and T-cells are well described in the art, in various texts and references. See, for example, Roitt's Essential Immunology. IM Roitt, PJ Delves. Oxford, Blackwell Science Publishers 2001

A cell-mediated response is an immune response that does not involve antibodies but rather involves the activation of macrophages, natural killer cells (NK), antigen-specific cytotoxic T-lymphocytes, and the release of various cytokines in response to an antigen. Cell-mediated immunity is used generally to refer to some Th cell activation, Tc cell activation and T-cell mediated responses. Cell mediated immunity is of particular importance in responding to viral infections.

For example, the induction of antigen specific CD8 positive T lymphocytes may be measured using an ELISPOT assay; stimulation of CD4 positive T-lymphocytes may be measured using a proliferation assay. Anti-influenza antibody titres may be quantified using an ELISA assay; isotypes of antigen-specific or cross reactive antibodies may also be measured using anti-isotype antibodies (e.g. anti -IgG, IgA, IgE or IgM)..Methods and techniques for performing such assays are well-known in the art.

Cytokine presence or levels may also be quantified. For example a T-helper cell response (Th1/Th2) will be characterized by the measurement of IFN-γ and IL-4 secreting cells using by ELISA (e.g. BD Biosciences OptEIA kits). Peripheral blood mononuclear cells (PBMC) or splenocytes obtained from a subject may be cultured, and the supernatant analyzed. T lymphocytes may also be quantified by fluorescence-activated cell sorting (FACS), using marker specific fluorescent labels and methods as are known in the art.

In one example of stimulation of an adaptive immune response, a dendritic cell may engulf an exogenous pathogen, or macromolecules comprising pathogen antigenic epitopes. The phagocytosed pathogen or macromolecules are processed by the cell, and smaller fragments (antigens) are displayed on the outer surface of the cell in the context of an MHC molecule. This MHC-antigen complex may subsequently be recognized by B- or T- cells. The recognition of the MHC-antigen complex by a B- or T-cell initiates a cascade of events, including clonal expansion of the particular lymphocyte, with an outcome being a specific, pathogen-directed immune response that kills cells infected with the pathogen. Aspects of the various events involved in the cascading immune response are known in the art, as may be found in Roitt, *supra.*

The term "subject" or "patient" generally refers to mammals and other animals including humans and other primates, companion animals, zoo, and farm animals, including, but not limited to, cats, dogs, rodents, rats, mice, hamsters, rabbits, horses, cows, sheep, pigs, elk or other ungulates, goats, poultry, etc.. The subject may have been previously assessed or diagnosed using other methods, such as those described herein or those in current clinical practice, or may be selected as part of a general population (a control subject).

In some embodiments, the present invention also provides for a composition for inducing an immune response in a subject. Compositions according to various embodiments of the invention may be used as a vaccine, or in the preparation of a vaccine.

The term 'vaccine' refers to an antigenic preparation that may be used to establish an immune response to a polypeptide, protein, glycoprotein, lipoprotein or other macromolecule. The immune response may be highly specific, for example directed to a single epitope comprising a portion of the macromolecule, or may be directed to several epitopes, one or more of which may comprise a portion of the macromolecule. Vaccines are frequently developed so as to direct the immune response to a pathogen. The immune response may be prophylactic, with the goal of preventing or ameliorating the effect of a future infection by a particular pathogen, or may be therapeutic, and administered with the goal of supplementing or stimulating a stronger immune response to one or more epitopes.

Several types of vaccines are known in the art. An inactivated vaccine is a vaccine comprising a previously killed pathogenic microorganism. Examples of killed vaccines include those for some influenza strains and hepatitis A live/attenuated vaccine comprises a non-killed pathogen that has been manipulated genetically, or grown under particular conditions, so that the virulence of the pathogen is reduced. Examples of live/attenuated vaccines include those for measles, mumps or rubella. A subunit vaccine is a vaccine comprising a fragment of the pathogenic microorganism. The fragment may include particular surface proteins or markers, or portions of surface proteins or markers, or other polypeptides that may be unique to the pathogen. Examples of subunit vaccines include vaccines include those described herein. Adjuvants, excipients, other additives for inclusion in a composition for use in a vaccine and methods of preparing such compositions will be known to those of skill in the art.

The terms 'peptide', 'polypeptide' and protein' may be used interchangeably, and refer to a macromolecule comprised of at least two amino acid residues covalently linked by peptide bonds or modified peptide bonds, for example peptide isosteres (modified peptide bonds) that may provide additional desired properties to the peptide, such as increased half-life. A peptide may comprise at least two amino acids. The amino acids comprising a peptide or protein described herein may also be modified either by natural processes, such as posttranslational processing, or by chemical modification techniques which are well known in the art. Modifications can occur anywhere in a peptide, including the peptide backbone, the amino acid side-chains and the amino or carboxyl termini. It is understood that the same type of modification may be present in the same or varying degrees at several sites in a given peptide.

Examples of modifications to peptides may include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer- RNA mediated addition of amino acids to proteins such as arginylation, and ubiquitination. See, for example, Wold F, Posttranslational Protein Modifications: Perspectives and Prospects, pgs. 1-12 in Posttranslational Covalent Modification of Proteins, B. C. Johnson, ed., Academic Press, New York, 1983; Seifter et al., Analysis for protein modifications and nonprotein cofactors, Meth. Enzymol. (1990) 182: 626-646 and Rattan et al. (1992), Protein Synthesis: Posttranslational Modifications and Aging," Ann NY Acad Sci 663: 48-62.

A substantially similar sequence is an amino acid sequence that differs from a reference sequence only by one or more conservative substitutions. Such a sequence may, for example, be functionally homologous to another substantially similar sequence. It will be appreciated by a person of skill in the art the aspects of the individual amino acids in a peptide of the invention that may be substituted.

Amino acid sequence similarity or identity may be computed by using the BLASTP and TBLASTN programs which employ the BLAST (basic local alignment search tool) 2.0 algorithm. Techniques for computing amino acid sequence similarity or identity are well known to those skilled in the art, and the use of the BLAST algorithm is described in ALTSCHUL et al. 1990, J Mol. Biol. 215: 403- 410 and ALTSCHUL et al. (1997), Nucleic Acids Res. 25: 3389-3402.

Standard reference works setting forth the general principles of peptide synthesis technology and methods known to those of skill in the art include, for example: Chan et al., Fmoc Solid Phase Peptide Synthesis, Oxford University Press, Oxford, United Kingdom, 2005; Peptide and Protein Drug Analysis, ed. Reid, R., Marcel Dekker, Inc., 2000; Epitope Mapping, ed. Westwood et al., Oxford University Press, Oxford, United Kingdom, 2000; Sambrook et al., Molecular Cloning: A Laboratory Manual, 3rd ed., Cold Spring Harbor Press, Cold Spring Harbor, NY 2001; and Ausubel et al., Current Protocols in Molecular Biology, Greene Publishing Associates and John Wiley & Sons, NY, 1994).

A protein or polypeptide, or fragment or portion of a protein or polypeptide is specifically identified when its sequence may be differentiated from others found in the same phylogenetic Species, Genus, Family or Order. Such differentiation may be identified by comparison of sequences. Comparisons of a sequence or sequences may be done using a BLAST algorithm (Altschul et al. 1009. J. Mol Biol 215:403-410). A BLAST search allows for comparison of a query sequence with a specific sequence or group of sequences, or with a larger library or database (e.g. GenBank or GenPept) of sequences, and identify not only sequences that exhibit 100% identity, but also those with lesser degrees of identity. For proteins with multiple isoforms, an isoform may be specifically identified when it is differentiated from other isoforms from the same or a different species, by specific detection of a structure, sequence or motif that is present on one isoform and is absent, or not detectable on one or more other isoforms.

It will be appreciated by a person of skill in the art that any numerical designations of amino acids within a sequence are relative to the specific sequence. Also, the same positions may be assigned different numerical designations depending on the way in which the sequence is numbered and the sequence chosen. Furthermore, sequence variations such as insertions or deletions, may change the relative position and subsequently the numerical designations of particular amino acids at and around a site or element of secondary or tertiary structure.

Nomenclature used to describe the peptides of the present invention follows the conventional practice where the amino group is presented to the left and the carboxy group to the right of each amino acid residue. In the sequences representing selected specific embodiments of the present invention, the amino- and carboxy-terminal groups, although not specifically shown, will be understood to be in the form they would assume at physiologic pH values, unless otherwise specified. In the amino acid structure formulae, each residue may be generally represented by a one-letter or three-letter designation, corresponding to the trivial name of the amino acid, such as is known in the art

Amino acids comprising the peptides described herein will be understood to be in the L- or D- configuration. In peptides and peptidomimetics of the present invention, D-amino acids may be substitutable for L-amino acids.

A peptidomimetic is a compound comprising non-peptidic structural elements that mimics the biological action of a parent peptide. A peptidomimetic may not have classical peptide characteristics such as an enzymatically scissile peptidic bond. A parent peptide may initially be identified as a binding sequence or phosphorylation site on a protein of interest, or may be a naturally occurring peptide, for example a peptide hormone. Assays to identify peptidomimetics may include a parent peptide as a positive control for comparison purposes, when screening a library, such as a peptidomimetic library. A peptidomimetic library is a library of compounds that may have biological activity similar to that of a parent peptide.

Amino acids may be substitutable, based on one or more similarities in the R-group or side-chain constituents, for example, hydropathic index, polarity, size, charge, electrophilic character, hydrophobicity and the like.

Peptides may include peptides comprising the amino acid sequences according to SEQ ID NOs: 10-17 and 19-32. Other peptides may include peptides having a substantially similar sequence to that of SEQ ID NOs: 10-17 and 19-32. Polypeptides may include polypeptides having a substantially similar sequence to that of amino acid permease, RplF, FabG, Aasf, PmpG-1, TC0420, Clp1, PmpE/F-2, Gap, or MOMP, or SEQ ID NO: 42, SEQ ID NO: 43, SEQ ID NO: 44, SEQ ID NO: 45, SEQ ID NO: 46 or SEQ ID NO: 47, , or fragments or portions thereof. Such peptides or proteins may be in isolation or in combination and may be linked to, or in combination with, tracer compounds, protein translocation sequences, liposomes, carbohydrate carriers, polymeric carriers or other agents or excipients as will be apparent to one of skill in the art.

It will be appreciated by a person of skill in the art that the numerical designations of the positions of amino acids within a sequence are relative to the specific sequence. Also the same positions may be assigned different numerical designations depending on the way in which the sequence is numbered and the sequence chosen. Furthermore, sequence variations such as insertions or deletions, may change the relative position and subsequently the numerical designations of particular amino acids at and around a site.

The adaptive immune response is exploited by vaccination to provide an immunological advantage to an otherwise naive subject. A vaccine may comprise immunogens that provide specific stimulation of an adaptive immune response to a virulent pathogen to which a subject has not yet been exposed.

An immunoproteomic approach to identifying candidate T-cell antigens may avoid the introduction of bias and maintain fidelity with antigen processing in a natural infection. An epitope that is never presented in the context of an MHC molecule will not be able to interact with immune effector cells such as T-cells or B-cells. On the other hand, an epitope identified by virtue of association with an MHC molecule may be able to interact with an immune effector cell, and thus have a greater likelihood of eliciting a suitable immune response.

Identification of an MHC-associated epitope from an antigen-presenting cell may be facilitated by enrichment of a cell lysate for MHC molecules, and release of peptides from the MHC complex. Methods of enriching a cell lysates for the MHC molecule fraction are known in the art and may include immunological methods such as immunoaffinity chromatography. Methods of releasing peptides from an MHC complex are known in the art and may include mild acidification of the lysate following enrichment. See, for example, Current Protocols in Immunology JE Coligan, ed. Wiley InterScience.

Identification of MHC-associated epitopes may be further facilitated by proteomics methods suited to analysis of minute quantities of proteins or peptides. Any given antigen presenting cell (APC) such as a dendritic cell (DC) may only 'present' one or two peptides in the MHC complexes. Further, ex vivo culture of an APC may be limited to the scale to which the APCs may be cultured. Sufficient sensitivity to enable analysis of femtomole-range concentration of peptides or proteins may be necessary. Fourier transform mass-spectrometry may provide such sensitivity. Examples of such mass spectrometers are known, and may include a linear ion trap-orbitrap (LTQ-Orbitrap) mass spectrometer (Makarov et al 2006. J. Am Soc Mass Spectrom 17:977-82), or a linear ion trap-Fourier Transform (LTQ-FT) mass spectrometer (deSouza et al 2006. 7:R72).

A schematic representation of an exemplary method involved in an immunoproteomics approach to identifying candidate T-cell antigens as described herein is shown in Figure 1.

Dendritic cells are isolated from a subject and co-incubated with an intracellular pathogen, for example *C. trachomatis* or *C. muridarum.* A preparation of bacterial LPS is included as a control. Following an incubation period, for example 24-48h, the dendritic cells are collected and lysed. Cells may be lysed by a variety of methods that preserve the MHC:antigen complex, for example sonication, lysis with mild detergents such as NP-40 or CHAPS, or with a hypotonic solution. Following cell lysis, MHC:antigen complexes may be isolated using immunogenic methods. For example, cellular debris following lysis is removed by centrifugation and the resulting supernatant comprising MHC:antigen complexes applied to an immunoaffinity column. The MHC:antigen complexes bound to the column are subsequently treated to release the antigen. For example, the column may be mildly acidified to selectively elute the antigens, leaving the MHC bound to the column. The column eluate may subsequently be concentrated by ultracentrifugation and applied to an reverse phase -HPLC, and as the antigens are eluted from the HPLC, the peptide sequence is determined by mass spectrometry.

Antigens found to associate with the MHC of dendritic cells may be identified in this manner, and such antigens may be immunogenic.

In order to further characterize a peptide or protein, nucleic acid encoding such a peptide or related proteins or fragments thereof may be cloned and expressed in a heterologous system. Methods of producing and manipulating such nucleic acids are known in the art, and are described in, for example Ausubel, et al., Current Protocols in Molecular Biology, John Wiley & Sons, New York, NY (1987-2006); or Sambrook et al., Molecular Cloning: A Laboratory Manual, 2nd edition, Cold Spring Harbour, NY (1989). Examples of such heterologous systems are known in the art and may include the pET system, a Baculovirus expression system, a yeast expression system, a mammalian expression system or the like. Alternatively, the peptides identified by the methods disclosed herein may be synthesized by chemical means that are known in the art.

The resulting peptide(s) or protein(s) may be, for example, exposed to cells cultured from a previously inoculated animal. The exposed cells may be assessed using an interferon gamma assay. Confirmation of the immunogenicity of the recombinant peptide(s) or protein(s) may be achieved by combining the recombinant peptide(s) or protein(s) with dendritic cells and T-cells in vitro. When the protein is processed by the dendritic cells and presented to the T-cells, an immunogenic protein will cause the T-cells to produce interferon gamma. The presence of interferon gamma in the supernatant confirms the immunogenicity of the protein or combination of proteins applied to the well. Examples of interferon assays are known in the art, and are described in, for example Rey-Ladino et al 2005 Infection and Immunity 73:1568-1577; Neild et al 2003. Immunity 18:813-823. It is within the ability of one of skill in the art to make any minor modifications to adapt such assays to a particular cellular model.

In another example, a candidate T cell antigen as described above may be used to inoculate a test subject, for example, an animal model of *Chlamydia* infection, such as a mouse. Methods of experimentally inoculating experimental animals are known in the art. For example, testing a *Chlamydia spp.* vaccine may involve infecting previously inoculated mice intranasally with an inoculum comprising an infectious *Chlamydia* strain, and assessing for development of pneumonia. An exemplary assay is described in, for example Tammiruusu et al 2007. Vaccine 25(2):283-290, or in Rey-Ladino et al 2005. Infection and Immunity 73:1568-1577. It is within the ability of one of skill in the art to make any minor modifications to adapt such an assay to a particular pathogen model.

In another example, testing a *Chlamydia* vaccine may involve serially inoculating female mice with a candidate T-cell antigen cloned and expressed as described above. A series of inoculations may comprise two, three or more serial inoculations. The candidate T-cell antigens may be combined with an adjuvant. About three weeks following the last inoculation in the series, mice are treated subcutaneously with 2.5 mg Depo-Provera and one week later both naive and immunized mice may be infected intravaginally with *Chlamydia.* The course of infection may be followed by monitoring the number of organisms shed at 2 to 7 day intervals for 6 weeks. The amount of organism shed may be determined by counting *Chlamydia* inclusion formation in Hela cells using appropriately diluted vaginal wash samples. Immunity may be measured by the reduction in the amount of organism shed in immunized mice compared to naive mice.

In another example, a combination of two, three, four or more candidate T-cell antigens may be co-inoculated in an experimental animal, or exposed to cells from an inoculated animal.

In another example, peptides comprising one, or more than one, of SEQ ID NO: 10, SEQ ID NO: 13, SEQ ID NO: 16, SEQ ID NO:17, SEQ ID NO: 20, SEQ ID NO: 21, SEQ ID NO: 22, SEQ ID NO: 23, SEQ ID NO: 27, SEQ ID NO: 28, SEQ ID NO: 31, PmpG-1, PmpE/F-2, SEQ ID NO: 42, SEQ ID NO: 43, SEQ ID NO: 45, SEQ ID NO: 46, and RplF may be used in a pharmaceutical preparation for inducing an immune response to one or more than one *Chlamydia* epitopes. The pharmaceutical preparation may be useful as a vaccine.

The pharmaceutical preparation may further comprise a polypeptide corresponding to one or more of SEQ ID NO: 44 or SEQ ID NO: 47.

In another example, a peptide may be used in the preparation of a medicament such as a vaccine composition, for the prevention or treatment of a *Chlamydia* infection. The peptide, or medicament or vaccine composition comprising the peptide, may be used for the prevention or treatment of a *Chlamydia* infection in a subject having, or suspected of having such a disease or disorder..

An "effective amount" of a peptide or polypeptide as used herein refers to the amount of peptide or polypeptide in the pharmaceutical composition to induce an immune response to a *Chlamydia* epitope in a subject. The effective amount may be calculated on a mass/mass basis (e.g. micrograms or milligrams per kilogram of subject), or may be calculated on a mass/volume basis (e.g. concentration, micrograms or milligrams per milliliter). Using a mass/volume unit, one or more peptides or polypeptides may be present at an amount from about 0.1 ug/ml to about 20 mg/ml, or any amount therebetween, for example 0.1, 0.5, 1, 2, 5, 10, 15, 20, 25, 30, 35, 40, 50, 60, 70, 80, 90, 100, 120, 140, 160 180, 200, 250, 500, 750, 1000, 1500, 2000, 5000, 10000, 20000 ug/ml, or any amount therebetween; or from about 1 ug/ml to about 2000 ug/ml, or any amount therebetween, for example 1.0, 2.0, 5.0, 10.0, 15.0, 20.0, 25.0, 30.0, 35.0, 40.0, 50.0 60.0, 70.0, 80.0, 90.0, 100, 120, 140, 160 180, 200, 250, 500, 750, 1000, 1500, 2000, ug/ml or any amount therebetween; or from about 10ug/ml to about 1000ug/ml or any amount therebetween, for example 10.0, 15.0, 20.0, 25.0, 30.0, 35.0, 40.0, 50.0 60.0, 70.0, 80.0, 90.0, 100, 120, 140, 160 180, 200, 250, 500, 750, 1000 ug/ml, or any amount therebetween; or from about 30ug/ml to about 1000ug/ml or any amount therebetween, for example 30.0, 35.0, 40.0, 50.0 60.0, 70.0, 80.0, 90.0, 100, 120, 140, 160 180, 200, 250, 500, 750, 1000 ug/ml.

Quantities and/or concentrations may be calculated on a mass/mass basis (e.g. micrograms or milligrams per kilogram of subject), or may be calculated on a mass/volume basis (e.g. concentration, micrograms or milligrams per milliliter). Using a mass/volume unit, one or more peptides or polypeptides may be present at an amount from about 0.1 ug/ml to about 20 mg/ml, or any amount therebetween, for example 0.1, 0.5, 1, 2, 5, 10, 15, 20, 25, 30, 35, 40, 50, 60, 70, 80, 90, 100, 120, 140, 160 180, 200, 250, 500, 750, 1000, 1500, 2000, 5000, 10000, 20000 ug/ml, or any amount therebetween; or from about 1 ug/ml to about 2000 ug/ml, or any amount therebetween, for example 1.0, 2.0, 5.0, 10.0, 15.0, 20.0, 25.0, 30.0, 35.0, 40.0, 50.0 60.0, 70.0, 80.0, 90.0, 100, 120, 140, 160 180, 200, 250, 500, 750, 1000, 1500, 2000, ug/ml or any amount therebetween; or from about 10ug/ml to about 1000ug/ml or any amount therebetween, for example 10.0, 15.0, 20.0, 25.0, 30.0, 35.0, 40.0, 50.0 60.0, 70.0, 80.0, 90.0, 100, 120, 140, 160 180, 200, 250, 500, 750, 1000 ug/ml, or any amount therebetween; or from about 30ug/ml to about 1000ug/ml or any amount therebetween, for example 30.0, 35.0, 40.0, 50.0 60.0, 70.0, 80.0, 90.0, 100, 120, 140, 160 180, 200, 250, 500, 750, 1000 ug/ml.

Compositions according to various embodiments of the invention, including therapeutic compositions, may be administered as a dose comprising an effective amount of one or more peptides or polypeptides. The dose may comprise from about 0.1 ug/kg to about 20mg/kg (based on the mass of the subject), for example 0.1, 0.5, 1, 2, 5, 10, 15, 20, 25, 30, 35, 40, 50, 60, 70, 80, 90, 100, 120, 140, 160 180, 200, 250, 500, 750, 1000, 1500, 2000, 5000, 10000, 20000 ug/kg, or any amount therebetween; or from about 1ug/kg to about 2000ug/kg or any amount therebetween, for example 1.0, 2.0, 5.0, 10.0, 15.0, 20.0, 25.0, 30.0, 35.0, 40.0, 50.0 60.0, 70.0, 80.0, 90.0, 100, 120, 140, 160 180, 200, 250, 500, 750, 1000, 1500, 2000 ug/kg, or any amount therebetween; or from about 10ug/kg to about 1000ug/kg or any amount therebetween, for example 10.0, 15.0, 20.0, 25.0, 30.0, 35.0, 40.0, 50.0 60.0, 70.0, 80.0, 90.0, 100, 120, 140, 160 180, 200, 250, 500, 750, 1000 ug/kg, or any amount therebetween; or from about 30ug/kg to about 1000ug/kg or any amount therebetween, for example 30.0, 35.0, 40.0, 50.0 60.0, 70.0, 80.0, 90.0, 100, 120, 140, 160 180, 200, 250, 500, 750, 1000 ug/kg.

One of skill in the art will be readily able to interconvert the units as necessary, given the mass of the subject, the concentration of the composition, individual components or combinations thereof, or volume of the composition, individual components or combinations thereof, into a format suitable for the desired application.

The amount of a composition administered, where it is administered, the method of administration and the timeframe over which it is administered may all contribute to the observed effect. As an example, a composition may be administered systemically e.g. intravenous administration and have a toxic or undesirable effect, while the same composition administered subcutaneously or intranasally may not yield the same undesirable effect. In some embodiments, localized stimulation of immune cells in the lymph nodes close to the site of subcutaneous injection may be advantageous, while a systemic immune stimulation may not.

Compositions according to various embodiments of the invention may be formulated with any of a variety of physiologically or pharmaceutically acceptable excipients, frequently in an aqueous vehicle such as Water for Injection, Ringer's lactate, isotonic saline or the like. Such excipients may include, for example, salts, buffers, antioxidants, complexing agents, tonicity agents, cryoprotectants, lyoprotectants, suspending agents, emulsifying agents, antimicrobial agents, preservatives, chelating agents, binding agents, surfactants, wetting agents, anti-adherents agents, disentegrants, coatings, glidants, deflocculating agents, anti-nucleating agents, surfactants, stabilizing agents, non-aqueous vehicles such as fixed oils, polymers or encapsulants for sustained or controlled release, ointment bases, fatty acids, cream bases, emollients, emulsifiers, thickeners, preservatives, solubilizing agents, humectants, water, alcohols or the like. See, for example, Berge et al. (1977. J. Pharm Sci. 66:1-19), or Remington- The Science and Practice of Pharmacy, 21st edition. Gennaro et al editors. Lippincott Williams & Wilkins Philadelphia.

Compositions comprising one or more peptides or polypeptides according to various embodiments of the invention may be administered by any of several routes, including, for example and without limitation, intrathecal administration, subcutaneous injection, intraperitoneal injection, intramuscular injection, intravenous injection, epidermal or transdermal administration, mucosal membrane administration, orally, nasally, rectally, topically or vaginally. See, for example, Remington- The Science and Practice of Pharmacy, 21st edition. Gennaro et al editors. Lippincott Williams & Wilkins Philadelphia. Carrier formulations may be selected or modified according to the route of administration.

Compositions according to various embodiments of the invention may be applied to epithelial surfaces. Some epithelial surfaces may comprise a mucosal membrane, for example buccal, gingival, nasal, tracheal, bronchial, gastrointestinal, rectal, urethral, vaginal, cervical, uterine and the like. Some epithelial surfaces may comprise keratinized cells, for example, skin, tongue, gingival, palate or the like.

Compositions according to various embodiments of the invention may be provided in a unit dosage form, or in a bulk form suitable for formulation or dilution at the point of use.

Compositions according to various embodiments of the invention may be administered to a subject in a single-dose, or in several doses administered over time. Dosage schedules may be dependent on, for example, the subject's condition, age, gender, weight, route of administration, formulation, or general health. Dosage schedules may be calculated from measurements of adsorption, distribution, metabolism, excretion and toxicity in a subject, or may be extrapolated from measurements on an experimental animal, such as a rat or mouse, for use in a human subject. Optimization of dosage and treatment regimens are discussed in, for example, Goodman & Gilman's The Pharmacological Basis of Therapeutics 11th edition. 2006. LL Brunton, editor. McGraw-Hill, New York, or Remington- The Science and Practice of Pharmacy, 21st edition. Gennaro et al editors. Lippincott Williams & Wilkins Philadelphia.

Compositions for use as vaccine compositions according to various embodiments of the invention may further comprise an adjuvant and administered as described. For example, a peptide or polypeptide for use in a vaccine composition may be combined with an adjuvant, examples of adjuvants include aluminum hydroxide, alum, Alhydrogel™ (aluminum trihydrate) or other aluminum-comprising salts, virosomes, nucleic acids comprising CpG motifs, squalene, oils, MF59, QS21, various saponins, virus-like particles, monophosphoryl-lipid A (MPL)/trehalose dicorynomycolate, toll-like receptor agonists, copolymers such as polyoxypropylene and polyoxyethylene, AbISCO, montanide ISA-51 or the like. In some embodiments, the one or more peptides or polypeptides may be combined with a cationic lipid delivery agent (Dimethyldioctadecylammonium Bromide (DDA) together with a modified mycobacterial cord factor trehalose 6,6'-dibehenate(TDB). Liposomes with or without incorporated MPL further been adsorbed to alum hydroxide may also be useful, see, for example US Patent Nos. 6,093,406 and 6,793,923 B2.

In the context of the present invention, the terms "treatment," , "treating", "therapeutic use," or "treatment regimen" as used herein may be used interchangeably are meant to encompass prophylactic, palliative, and therapeutic modalities of administration of the compositions of the present invention, and include any and all uses of the presently claimed compounds that remedy a disease state, condition, symptom, sign, or disorder caused by an inflammation-based pathology, infectious disease, allergic response, hyperimmune response, or other disease or disorder to be treated, or which prevents, hinders, retards, or reverses the progression of symptoms, signs, conditions, or disorders associated therewith.

Standard reference works setting forth the general principles of immunology known to those of skill in the art include, for example: Harlow and Lane, Antibodies: A Laboratory Manual, 2d Ed., Cold Spring Harbor Laboratory Press, Cold Spring Harbor, N. Y. (1999); Harlow and Lane, Using Antibodies: A Laboratory Manual. Cold Spring Harbor Laboratory Press, New York; Coligan et al. eds. Current Protocols in Immunology, John Wiley & Sons, New York, NY (1992-2006); and Roitt et al., Immunology, 3d Ed., Mosby-Year Book Europe Limited, London (1993).

Design and selection of primers for PCR amplification of sequences will readily be apparent to those of skill in the art when provided with one or more nucleic acid sequences comprising the sequence to be amplified. Selection of such a sequence may entail determining the nucleotide sequence encoding a desired polypeptide, including initiation and termination signals and codons. A skilled worker, when provided with the nucleic acid sequence, or a polypeptide sequence encoded by the desired nucleic acid sequence, will be able to ascertain one or more suitable segments of the nucleic acid to be amplified, and select primers or other tools accordingly. Standard reference works setting forth the general principles of recombinant DNA technology known to those of skill in the art include, for example: Ausubel et al, Current Protocols In Molecular Biology, John Wiley & Sons, New York (1998 and Supplements to 2001); Sambrook et al, Molecular Cloning: A Laboratory Manual, 2d Ed., Cold Spring Harbor Laboratory Press, Plainview, New York (1989); Kaufman et al , Eds., Handbook Of Molecular And Cellular Methods In Biology And Medicine, CRC Press, Boca Raton (1995); McPherson, Ed., Directed Mutagenesis: A Practical Approach, IRL Press, Oxford (1991).

**Alternate examples:** An alternative method to generate MHC-bound peptides for subsequent analysis using the mass spectrophotometric methods described herein includes use of an immortalized DC line from C57BL/6 mice transfected with myc and ras oncogenes that are immunologically equivalent to primary DCs, expressing high levels of MHC (Shen Z 1997 J. Immunol 158:2723-2730). Such immortalized dendritic cells may be exposed to proteins or peptides having *Chlamydia*l epitopes, and used in the methods described herein.

Compositions may be used as vaccine formulations and tested in nonhuman primates at a suitable facility, such as the University of Washington's Primate Centre. Groups of suitable primates, e.g. Cynomolgus macaques, may be immunized with adjuvant alone as negative control, PmpG or SEQ ID NO: 42, PmpF or SEQ ID NO: 43, MOMP or SEQ ID NO: 44, or combinations thereof with an adjuvant or PmpG or SEQ ID NO: 42, PmpF or SEQ ID NO: 43, and MOMP or SEQ ID NO: 44 pooled and combined with an adjuvant. The compositions may be administered to the subjects by injection (e.g. intramuscular injection) with an effective dose (e.g. 100 micrograms per antigen at day 0 and months 1 and 3). Following the administration schedule, at four months the subjects may be challenged intracervically with lOX 1050 serovar 0 C. *trachomatis* and followed at weekly intervals with quantitative cultures and NAAT tests for four months or until clearing occurs. At eight months after the initial administration, the animals may be examined by laparoscopy (or ultrasound or MRI) to visually define the upper genital tract pathology. Serum and peripheral blood cells may be collected at baseline, 1, 3, and 4 though 8 months and prior to Imaging.

### Articles of Manufacture

In an example, an article of manufacture is provided comprising packaging material and a composition comprising one or more peptides or polypeptides as provided herein. The composition includes a physiologically or pharmaceutically acceptable excipient, and may further include an adjuvant, a delivery agent, or an adjuvant and a delivery agent, and the packaging material may include a label which indicates the active ingredients of the composition (e.g. the peptide or polypeptide, adjuvant or delivery agent as present). The label may further include an intended use of the composition, for example as a therapeutic or prophylactic composition to be used in the manner described herein.

### Kits

In another example, a kit for the preparation of a medicament, comprising a composition comprising one or more peptides as provided herein, along with instructions for its use is provided. The instructions may comprise a series of steps for the preparation of the medicament, the medicament being useful for inducing a therapeutic or prophylactic immune response in a subject to whom it is administered. The kit may further comprise instructions for use of the medicament in treatment for treatment, prevention or amelioration of one or more symptoms of a *Chlamydia* infection, and include, for example, dose concentrations, dose intervals, preferred administration methods or the like.

The present invention will be further illustrated in the following examples. However it is to be understood that these examples are for illustrative purposes only, and should not be used to limit the scope of the present invention in any manner.

### Methods

**Mice:** Female C57BL/6 mice (8 to 10 weeks old) were purchased from Charles River Canada (Saint Constant, Canada).

**Dendritic cell generation from bone marrow :** Dendritic cells (DCs) were generated following the protocol described by Lutz et al. 1999 J Immunol Methods 223:77-92. Briefly, bone marrow cells were prepared from the femora and tibiae of naive C57BL/6 mice and cultured in DC medium. DC medium is Iscove's modified Dulbecco's medium (IMDM) supplemented with 10% FCS, 0.5 mM 2-ME, 4 mM L-glutamine, 50 µg/ml gentamicin, 5% of culture supernatant of murine GM-CSF transfected plamacytoma X63-Ag8 and 5% of culture supernatant of murine IL-4 transfected plamacytoma X63-Ag8 which contained approximately 10 ng/ml of GM-CSF and 10 ng/ml of IL-4 respectively. Culture medium was changed every three days.

**Infection of dendritic cells and purification of MHC-bound peptides:** A total of 4x10⁹ immature bone-marrow derived DCs were used for each experiment. Briefly, DCs were infected with *C. muridarum* at a 1:1 multiplicity of infection for 24 hr. As a control, DCs were incubated with LPS (1 microgram/ml; Sigma)

DCs treated with *C. muridarum* or LPS (as a control) were solubilized in lysis buffer [1% CHAPS, 150mM NaCl, 20 mM Tris-HCl pH 8, 0.04% Sodium azide]. Protease inhibitors (Sigma) were added to minimize peptide degradation. MHC molecules (class I and class II) from *Chlamydia*-loaded and LPS-treated DCs were isolated using allele-specific anti-MHC monoclonal affinity columns (Table 1). The purified MHC molecules were washed and the peptides were eluted with 0.2N acetic acid and separated from high molecular weight material by ultrafiltration through 5-kDa cut-off membrane (Cox et al. 1997. The application of mass spectrometry to the analysis of peptides bound to MHC molecules in MHC- A practical Approach, pp. 142-160).

**Identification of MHC-bound peptides:** The purified MHC-bound peptides were analyzed using a linear trapping quadrupole/Fourier transform ion cyclotron resonance mass spectrometer (LTQ-FT, Thermo Electron) on-line coupled to Agilent 1100 Series nanoflow HPLCs using nanospray ionization sources (Proxeon Biosystems, Odense, Denmark). Analytical columns were packed into 15 cm long, 75 mm inner diameter fused silica emitters (8 mm diameter opening, pulled on a P-2000 laser puller from Sutter Instruments) using 3 mm diameter ReproSil Pur C₁₈ beads. LC buffer A consisted of 0.5% acetic acid and buffer B consisted of 0.5% acetic acid and 80% acetonitrile. Gradients were run from 6% B to 30% B over 60 minutes, then 30% B to 80% B in the next 10 minutes, held at 80% B for five minutes and then dropped to 6% B for another 15 minutes to recondition the column. The LTQ-FT was set to acquire a full range scan at 25,000 resolution in the FT, from which the three most intense multiply-charged ions per cycle were isolated for fragmentation in the LTQ. At the same time selected ion monitoring scans in the FT were carried out on each of the same three precursor ions. Fragment spectra were extracted using DTASuperCharge (available online from MSQuant Sourceforge - http://msquant.sourceforge.net and described in Mortensen et al 2009 J. Proteome Research 9:393-403) and searched using the Mascot algorithm against a database comprised of the protein sequences from mouse (self) and *Chlamydia.*

**Table 1: Anti-MHC Monoclonal Antibodies Used for MHC Purification.**

| **MHC type** | **mAb Designation** | **ATCC#** |
|---|---|---|
| Class I: H-2K^{b} | AF6-88.5.3 | HB-158 |
| Class I: H-2D^{b} | 20-80-4S * | HB-11 |
| Class II: I-A^{b} | Y-3P | HB-183 |

**Interferon (IFN)-gamma assay of T-cell response to specific peptides:** The *Chlamydia* peptides that associated with the class II MHC molecules were examined for recognition by *Chlamydia* specific CD4 T cells *in vitro.* Peptides corresponding to the sequences of each of the eight class II epitopes (SEQ ID NOs: 10-17) were synthesized and purified to 54-74% (Sigma Corporation) and then resolubilized in dimethyl sulfoxide at a concentration of 4 mg/mL. Immature DCs were generated and following maturation with LPS (1 microgram/ml), DCs were incubated for 4 hr with 10 microgram/ml peptide. *Chlamydia*-specific CD4 T cells were generated by infecting C57BL/6 mice with *C. muridarum* as described in Rey-Ladino et al. 2005. Infec Immun 73:1568-1577. Briefly, spleens were isolated from naive or mice that had cleared a *C. muridarum* infection, and CD4+ T cells were isolated with a MACS CD4+T-cell isolation kit (Miltenyi Biotech). Peptide-pulsed DCs and CD4 T cells were co-cultured at a ratio of 1:3 and IFN-gamma production was determined from the culture supernatant following 48 hr incubation by ELISA (Pharmingen) as described (Rey-Ladino et al. 2005. Infec Immun 73:1568-1577). The amount of IFN-gamma present in the supernatants was used as a measure of antigen-specific T-cell recognition.

**Delivery of *Chlamydia* MHC class II binding peptides by ex vivo pulsed DCs :** The peptides (SEQ ID NOs: 10-17) derived from the *Chlamydia*l proteins (PmpG, PmpF, L6 ribosomal protein, 3-oxoacyl-(acyl carrier protein) reductase, glyceraldehydes-3-phosphate dehydrogenase, ATP-dependent Clp protease, anti-anti-sigma factor the hypothetical protein TC0420) were pooled and *used* to pulse LPS-matured BMDCs for 4 h at 37°C. The peptide-pulsed DCs were washed three times and 1×10⁶ cells were adoptively transferred intravenously to naive C57BL/6 mice and this process was repeated one week later. As a control, one group of mice received LPS-matured DCs that had not been treated with peptides (DC alone). One week following the final adoptive transfer, the mice were infected intranasally with 2000 IFU of *C. muridarum* and body weight was monitored every 48 hours post-infection.

***Chlamydia* strains:** *C. muridarum* strain Nigg (mouse pneumonitis strain) was cultured in Hela 229 cells and elementary bodies (EBs) were purified by discontinuous density gradient centrifugation and stored at -80°C as previously described in Hansen et al. 2008 J Infect Dis 198:758-767. The infectivity of purified EBs was titrated by counting *Chlamydia* inclusion forming units (IFUs) on HeLa cell monolayer with anti-EB mouse polyclonal antibody followed by biotinylated anti-mouse IgG (Jackson ImmunoResearch) and a DAB substrate (Vector Laboratories).

**Cloning the *Chlamydia*l protein antigens :** The proteins containing the MHC II binding *Chlamydia* peptides (SEQ ID NOs: 10-17) were cloned, expressed and purified as follows: *rplF, fabG, aasf, pmpG-1, TC0420, clp-1, pmpE*/*F-2* and *gap* DNA fragments were generated by PCR using genomic DNA isolated from *C. muridarum.* The PCR products were purified and cloned into either pGEX-6P-3 (GE Healthcare) for *rplF, fabG, aasf, TC0420,* and *clp-1* or pET32a (Novagen) for *pmpG-1, pmpE*/*F-2* and *gap* after restriction enzyme digestion with *BamH*I / *Not*I using standard molecular biology techniques. For *pmpG-1, pmpE*/*F-2,* only the first half of the gene (*pmpG-1*₂₅₋₅₀₀*, pmpE*/*F2₂₅₋₅₇₅*encoding amino acids 25 - 500 and 25-575 of PmpG-1 and PmpE/F-2, respectively) was cloned into the vector for expression. The sequences of the sub-cloned genes were confirmed by sequencing with dye-labeled terminators using the ABI PRISM kit (PE Biosystems). Plasmids containing the *rplF, fabG, aasf, pmpG-1*₂₅₋₅₀₀*, TC0420, clp-1, pmpE*/*F-2₂₅₋₅₇₅* and *gap* sequences were transformed into the *E. coli* strain BL21(DE3) (Stratagene) where protein expression was carried out by inducing the *lac* promoter for expression of T7 RNA polymerase using isopropyl-beta-D-thiogalactopyranoside. The expressed RplF, FabG, Aasf, TC0420, and Clp-1 proteins with N-terminal GST-tag were purified from *E. coli* lysates by affinity chromatography using glutathione sepharose 4 fastflow purification system (GE Healthcare). PmpG-1₂₅₋₅₀₀, PmpE/F-2₂₅₋₅₇₅ and Gap proteins with N-terminal His-tag were purified by nickel column using the His bind purification system (Qiagen). LPS removal was carried out by adding 0.1 % Triton-114 in the wash buffers during purification.

**Transfection of dendritic cells with *Chlamydia* proteins:** After an 8-day culture, dendritic cells (DCs) were harvested for transfection with *Chlamydia* protein antigens. Approximately 65∼70% percent of the cell preparation were DCs as judged by a staining with anti-CD11c monoclonal antibody. DCs harvested on day 8 were washed twice in RPMI 1640. Sixty microlitres of the liposomal transfection reagent N-[1-(2,3-dioleoyloxy)propyl]-N,N,N-trimethylammonium methyl-sulfate (DOTAP; Roche) and individual or combination of *Chlamydia* proteins PmpG-1₂₅₋₅₀₀ (amino acids 25-500 of PmpG-1), RplF, PmpE/F-2₂₅₋₅₇₅ (amino acids 25-575 of PmpE/F-2), MOMP or the negative control protein, GST were mixed with 240 µl RPMI 1640 at room temperature in polystyrene tubes for 20 min. The final concentration of PmpG-1₂₅₋₅₀₀, PmpE/F-2₂₅₋₅₇₅, MOMP or GST protein in the DOTAP/protein mixtures is 0.2 mg/ml and RplF protein is 0.8 mg/ml. DCs (2∼3 x10⁷) in 3 ml RPMI 1640 were added to the DOTAP/protein mixtures. The protein-transfected DCs were incubated for 3 h at 37 °C, washed twice, resuspended in DC medium and then cultured overnight in the presence of 0.25 µg /ml LPS for maturation. DCs on day 8 pulsed with live EB (MOI:1) for 24 hours was prepared as a positive control. Antigen loaded DCs were used for *in vitro* immunohistochemical analysis and *in vivo* immunization.

**Immunohistochemistry:** The protein-transfected DCs were deposited onto Micro Slides using Shandon Cytospin (Thermo Electron Corp.). The DCs on the slides were fixed for 20 min in 4 % paraformaldehyde in PBS. Subsequently, they were permeabilized for 10 min in 0.5 % Triton X-100 in PBS. The cells were blocked for 20 min with PBS containing 1 % horse serum, and incubated with corresponding antigen-specific polyclonal murine serum (1: 200) respectively for 2 h. All anti*-Chlamydia* protein polyclonal antibodies (PmpG-1₂₅₋₅₀₀, RplF, PmpE/F-2₂₅₋₅₇₅, or MOMP) were made in our laboratory as follows: Balb/c mice were immunized three times subcutaneously with 10 µg recombinant *Chlamydia* protein formulated with Incomplete Freunds Adjuvant (Sigma). Two weeks after the final immunization, sera from each group were collected and pooled. All anti*-Chlamydia* protein polyclonal antibodies had titers ≥1: 500,000 dilution as determined by ELISA. Biotinylated horse anti-mouse IgG (1: 2000) (Vector Laboratories) was added and then the cells were incubated again for 1 h. Finally, the cells were incubated for 45 min with ABC Reagent (Vector Laboratories) and incubated with peroxidase substrate solution (DAB substrate kit SK-4100; Vector Laboratories) until the desired stain intensity developed. The slides were rinsed in tap water, counterstained with 0.1 % toluidine blue, and again rinsed in tap water. All incubations were performed at room temperature and the slides were washed in PBS three times between incubations.

**ELISA:** CD4 T cells were isolated from the spleens of mice immunized i.p. with *Chlamydia* (14) or naive mice using MACS CD4 T cell isolation kit (Miltenyi Biotec). CD4 T cells of at least 90% purity were obtained as measured by FACS (data not shown). Purified BMDCs were cultured in a 96-well plate at 2 x10⁵ cells/well and matured with LPS (1 microgram/ml) overnight, followed by treatment with 2 microgram/ml *Chlamydia* peptides or control peptides for 4 h, at which point the cells were washed to remove unbound peptides. After a 48-h coculture with CD4 T cells (5 x 10⁵/well), supernatants were collected and the production of IFN-gamma in the supernatants was determined by ELISA as described in Rey-Ladino et al., 2005 (supra).

**ELISPOT assay:** For the IFN-gamma ELISPOT assay, 96-well MultiScreen-HA filtration plates (Millipore) were coated overnight at 4 C with 2 µg/ml of murine IFN-gamma specific monoclonal antibody (BD PharMingen, Clone R4-6A2). Splenocytes isolated from mice in AIM-V medium were added to the coated plates at 10⁶ cells per well in presence of individual *Chlamydia* peptide at 2 µg/ml or individual *Chlamydia* protein at 1 µg/ml. After 20 h incubation at 37 °C and 5% CO₂, the plates were washed and then incubated with biotinylated murine IFN-gamma specific monoclonal antibodies (BD PharMingen, Clone XMG1.2) at 2 µg/ml. This was followed by incubation with streptavidin-alkaline phosphatase (BD PharMingen) at a 1:1000 dilution. The spots were visualized with a substrate consisting of 5-bromo-4-chloro-3-indolyl phosphate and nitro blue tetrazolium (Sigma-Aldrich).

**Adoptive transfer of DCs transfected with *Chlamydia* protein antigens:** Mice were vaccinated three times with a 2-week interval, intravenously (*i*.*v*.) into the tail vein with 1 × 10⁶ DCs transfected with *Chlamydia* protein PmpG-1₂₅₋₅₀₀, RplF, PmpE/F-2₂₅₋₅₇₅ or MOMP in 200 µl of PBS. DCs pulsed with live EB or with GST protein were used as positive and negative controls, respectively. Two weeks after the last immunization, six mice of each group were euthanized to isolate splenocytes for IFN-gamma ELISPOT assay. The remaining mice were used for *Chlamydia* infection challenge.

**Pulmonary and cervicovaginal challenge and determination of *Chlamydia*** titers: Two weeks after the final immunization, five to ten mice from each group were intranasally challenged with 2000 IFU of *C. muridarum.* Weight loss was monitored each or every two days. On 10 day after intranasal challenge, the mice were euthanized and the lungs were collected for *Chlamydia* titration. Single-cell suspensions were prepared by homogenizing the lungs with tissue grinders and coarse tissue debris was removed by centrifugation at 1000 ×g for 10 min at 4°C. The clarified suspensions were serially diluted and immediately inoculated onto HeLa 229 monolayers for titration. For genital tract infections, one week after the final immunization, ten mice from each group were injected subcutaneously with 2.5 mg of medroxyprogesterone acetate (Depo-Provera; Pharmacia and Upjohn). One week after Depo-Provera treatment, the mice were challenged intravaginally with 1500 IFU of *C. muridarum.* Cervicovaginal washes were taken at day 6 and day 13 after infection and stored at -80°C for titration on HeLa cells as described previously in Bilenki et al. 2005 J Immunol 175:3197-3206.

**Adjuvants:** Three adjuvants (CpG ODN 1826, AbISCO-100, and Dimethyldioctadecylammonium Bromide/D-(+)-trehalose 6,6'-dibenhate (DDA/TDB) were studied in the present study. CpG ODN 1826 (5'-TCCATGACGTTCCTGACGTT-3', phosphorothioate modified, Integrated DNA Technologies, Inc.) (SEQ ID NO: 48) was used as either a free form (Free CpG) or a form conjugated with liposomal nanoparticle (LN-CpG). AbISCO-100 adjuvant (ISCONOVA Sweden) is a selection of purified fractions of quillaja saponins formulated with a mixture of cholesterol (ovine wool) and phosphatidyl choline (egg). DDA Dimethyldioctadecylammonium Bromide (product No.890810P) and TDB D-(+)-trehalose 6,6'-dibehenate (product No. 890808P) were purchased from Avanti Polar Lipids (Alabaster AL). For DDA/TDB formulation, DDA was mixed into 10mM Tris-buffer at pH 7.4 to a concentration of 1.67 mg/ml, heated to 80 °C while being stirred continuously on a magnetic hot plate for 20 min, and then cooled to room temperature. TDB was suspended in dH₂O containing 2% dimethyl sulfoxide to a concentration of 5 mg/ml by rep eated passaging through a fine-tipped pipette followed by 30 seconds of vortexing. This step was repeated three times before freezing the solution at -20°C until use. 5ml TDB (1 mg/ml) was added into 15ml DDA (1.67 mg/ml). The resulting solution was then vortexed briefly and stored at 4 °C until use. The final concentration of DDA was 1.25 mg/ml and TDB was 0.25 mg/ml. Each inoculation dose, 200 µl for immunization contained 250 µg DDA and 50 µg TDB.

**Mouse immunization:** Four mouse trials were conducted in this study. All mice except the live EB group were immunized three times subcutaneously (sc) in the base of tail at 2 week intervals. Mice intranasally infected with 1500 inclusion-forming units (IFU) live C. *muridarum* (EB) were set up as positive controls.

In the first trial, groups of six C57/BL6 mice were immunized with 20 µg *Chlamydia* protein (PmpG-1 or MOMP) mixed with 700 µg LN-CpG or 700 µg free CpG. Groups of LN-CpG alone and PBS immunization were set up as negative controls. In the second trial, groups of eight C57/BL6 mice were immunized with 5 µg individual *Chlamydia* proteins PmpG-1, PmpE/F-2, MOMP or a combination (1.67 µg for each protein) formulated with AbISCO-100 (12µg) or DDA/TDB (250 µg DDA,50 µg TDB) as follows: (1) PmpG-1+AbISCO-100 (PmpG+AbISCO); (2) PmpE/F-2+AbISCO-100 (PmpF+AbISCO); (3) MOMP+AbISCO-100 (MOMP+AbISCO); (4) PmpG-1+PmpE/F-2+MOMP +AbISCO-100 (G+F+M+AbISCO); (5) AbISCO-100 alone (AbISCO alone); (6) PmpG-1+DDA/TDB (PmpG+DDA/TDB); (7) PmpE/F-2+DDA/TDB (PmpF+DDA/TDB); (8) MOMP+DDA/TDB; (9) PmpG-1+ PmpE/F-2+MOMP +DDA/TDB (G+F+M+DDA/TDB); (10) DDA/TDB alone; (11) PBS; or (12) EB. In the third trial, three groups of eight BALB/c mice were immunized as follows: (1) G+F+M+DDA/TDB; (2) DDA/TDB alone; (3) EB. The mice in above three animal trials were then challenged with live EB for protection and pathology evaluation.

In the fourth trial, groups of sixteen C57/BL6 mice were immunized with 5 µg PmpG-1 formulated with DDA/TDB (250 µg DDA, 50 µg TDB), AbISCO-100 (12µg) or CpG (20 µg). Two weeks after the last immunization, half of the mice in each group were sacrificed to isolate splenocytes for lymphocyte multi-color flow cytometry, ELISA and enzyme-linked immunospot (ELISPOT) assays; the other half of the mice were challenged with live EB and sacrificed seven days later to isolate splenocytes and iliac lymph nodes for multi-color flow cytometry.

**Genital tract infection and determination of *Chlamydia* titer:** One week after the last immunization, mice were injected s.c. with 2.5 mg of medroxyprogesterone acetate (Depo-Provera; Pharmacia and Upjohn). One week after Depo-Provera treatment, mice were challenged intravaginally with 1500 IFU of *C. muridarum.* Cervicovaginal washes were taken atselected dates after infection and stored at -80°C for titration on HeLa cells as described (Bilenki et al., 2005. J. Immunol 175:3197-3206).

**Cytokine measurement:** The culture supernatants of the splenocytes stimulated with PmpG-1 protein or HK-EB for 48 hours were collected and analyzed with respect to TNF-α production with a sandwich ELISA using corresponding specific capture and detection antibodies (BD PharMingen). TNF-α levels were calculated using standard curve constructed by recombinant murine TNF-α (BD PharMingen).

**Multiparameter flow cytometry:** Two weeks after the last immunization or seven days after live EB challenge, the mice from specified groups were sacrificed and the cells harvested from spleen and iliac lymph nodes (after challenge) were stimulated with 2 µg/ml antibody to CD28 and PmpG-1 protein (1 µg/ml) or HK-EB (5×105IFU/ml) in complete RPMI 1640 for 4 h at 37°C. Brefeldin A was added at a final concentration of 1 µg/ml and cells were incubated for an additional 12 h before intracellular cytokine staining. Cells were surface stained for CD3, CD4 and CD8 as well as the viability dye, red-fluorescent reactive dye (RViD) (L23102, Molecular Probes) followed by staining for IFN-γ, TNF-α and IL-17 using the BD Cytoperm kit according to the manufacturer's instruction. Finally, the cells were resuspended in 4% formaldehyde solution. All antibodies and all reagents for intracellular cytokine staining were purchased from BD Pharmingen except where noted. We acquired 200,000 live lymphocytes per sample using an Aria flow cytometer and analyzed the data using FlowJo software (Tree Star).

**Evaluation of mouse genital tract tissue pathologies:** Mice were sacrificed 60 days after challenge and the mouse genital tract tissues were isolated. Hydrosalpinx in only one (unilateral) or both (bilateral) oviducts were visually identified as the pathologic outcome in the vaccine groups.

**Statistical analysis:** All data were analyzed with the aid of a software program (GraphPad Prism 3.0). Differences between the means of experimental groups were analyzed using an independent, two-tailed *t*-test at the level of *p* < 0.05.

### Example 1: Identification of MHC class II (I-Ab)-bound Chlamydial peptides

The purified MHC-bound peptides were identified by tandem mass spectrometry. In total 318 MHC Class II (I-Ab)-bound peptides were isolated. Many of these peptides were derived from the same epitope, with varying degrees of proteolytic processing and 157 distinct epitopes were isolated from 137 unique source proteins. As determined by BLAST identification of the peptides using the National Centre for Biotechnology Information database (GenPept), four peptides were derived from the *Chlamydia* L6 ribosomal protein (RplF), two peptides from the 3-oxoacyl-(acyl carrier protein) reductase, two peptides from polymorphic membrane protein G (PmpG), one peptide from polymorphic membrane protein E/F (PmpE/F), one peptide from glyceraldehydes-3-phosphate dehydrogenase, one peptide from ATP-dependent Clp protease, one peptide from the anti-anti-sigma factor and one peptide from a hypothetical protein TC0420, all from the *C. muridarum* proteome (Table 2).

### Example 2: Identification of MHC Class I (H2-Kb)-bound peptides

One of the H2-Kb-bound peptides that was isolated (SEQ ID NO: 19), corresponded to an amino acid permease from the *C. muridarum* proteome. The 79 remaining peptides that were isolated with were self-peptides.

**Table 2: MHC-bound peptides**

| | **Protein** | **Peptide sequence** | ***C.trachomatis* Accession** |
|---|---|---|---|
| 19 | Amino acid permease | SSLFLVKL | NP_219919 |
| 20 | Ribosomal protein L6 | GNEV**FVSPAAHII**D | AAC68115 |
| 21 | Ribosomal protein L6 | GNEV**FVSPAAHII**DRPG | AAC68115 |
| 22 | Ribosomal protein L6 | KGNEV**FVSPAAHII**DRPG | AAC68115 |
| 23 | Ribosomal protein L6 | EV**FVSPAAHII**DRPG | AAC68115 |
| 24 | 3-oxoacyl-(acyl carrier protein) reductase | SPGQTN**YAAAKAGII**G | NP_219742 |
| 25 | 3-oxoacyl-(acyl carrier protein) reductase | SPGQTN**YAAAKAGII**GFS | NP_219742 |
| 26 | Anti-anti-sigma factor | KLDG**VSSPAVQES**ISE | NP_219936 |
| 27 | Polymorphic membrane protein G1 family | SPI**YVDPAAAGG**QPPA | AAC68469 |
| 28 | Polymorphic membrane protein G1 family | ASPI**YVDPAAAGG**QPPA | AAC68469 |
| 29 | Hypothetical protein (CT143) | DLN**VTGPKIQTD**VD | NP_219646 |
| 30 | ATP-dependent Clp protease | IGQE**ITEPLANTV**IA | NP_220225 |
| 31 | Polymorphic membrane protein E/F family F-2 | FHL**FASPAANYI**HTGP | AAC68468 |
| 32 | Glyceraldehyde 3-phosphate dehydrogenase | MTT**VHAATATQS**VVD | NP_220020 |

### Example 3 : Recognition of Chlamydial peptides and production of interferon gamma by immune CD4+ T-cells.

Peptides comprising the identified MHC class II *Chlamydia* epitopes were synthesized (SEQ ID NOs: 10-17) and examined for recognition by *Chlamydia* specific CD4⁺ T cells *in vitro.* Briefly, CD4⁺ T cells from immune or naive mice were co-cultured with peptide-pulsed DCs as described (Cohen et al., 2006. Journal of Immunology 176: 5401-5408). IFN-gamma production was determined from the culture supernatant following 48 hr incubation by ELISA. All the MHC class II *Chlamydia* peptides were recognized by *Chlamydia*-specific CD4 T cells as measured by antigen specific IFN-gamma production (Figure 2), suggesting that these antigens are immunologically relevant and may be useful as antigens in *Chlamydia* vaccine development.

### Example 4: Delivery of Chlamydia MHC class II binding peptides by ex vivo pulsed DCs

To evaluate whether the identified *Chlamydia* MHC class II peptides (SEQ ID NOs: 10-17) were able to protect mice against *Chlamydia* infection using a lung infection model, the peptides (SEQ ID NOs: 10-17) were synthesized, pooled together and used to load LPS-matured DCs *ex vivo.* The peptide-pulsed DCs were adoptively transferred intravenously to naive C57BL/6 mice. As a control, another group of mice received LPS-matured DCs that had not been treated with peptides (DC alone). One week following the second adoptive transfer both groups of mice were infected intranasally with 2000 inclusion forming units (IFUs) of *C. muridarum.* Body weight was monitored every 48 hours post infection. Mice adoptively transferred with peptide-pulsed DCs (Figure 3) reversed body weight loss by day 10 post-infection returning to their pre-infection body weight by day 15. In contrast, mice that had been adoptively transferred with LPS-matured non pulsed DCs failed to regain their starting body weight over this time.

### Example 5: Identification of the immunodominant Chlamydia antigens among the 8 MHC class II binding peptides

To determine which individual peptides or proteins are immunodominant in the context of natural infection, we performed IFN-gamma ELISPOT assays using splenocytes from C57BL/6 mice that had recovered from live *C. muridarum* infection. Splenocytes from mice harvested one month after *C. muridarum* infection were stimulated *in vitro* for 20 h with either 2 µg/ml of the individual peptide or pooled peptide epitopes (SEQ ID NOs: 10-17) or 1 µg/ml of the individual protein or with pooled proteins (RplF, FabG, Aasf, PmpG-1, TC0420,Clp, PmpE/F and Gap). Irrelevant OVA peptide and GST were used as peptide and protein negative controls respectively and heat killed EB (HK-EB) as positive control. Since MOMP has been long standing candidate in *Chlamydia* vaccine studies, MOMP was also set up as a reference antigen. As shown in Figure 4, immune splenocytes exposed to HK-EB developed the highest numbers of IFN-gamma secreting cells where more than 1000 IFN-gamma-secreting cells were detected among 10⁶ splenocytes. In contrast, splenocytes stimulated with the OVA peptide or GST protein as negative controls showed nearly blank background levels indicating that IFN-gamma secreting cells detected in the experimental system are *Chlamydia* antigen-specific. Stimulation by pooled peptides or pooled proteins induced significantly higher numbers of IFN-gamma secreting cells than stimulation with individual *Chlamydia* antigens (*p* < 0.05).

Immune splenocytes stimulated with individual *Chlamydia* antigens exhibited markedly different levels of IFN-gamma response (Figure 4). The results demonstrated that IFN-gamma responses in immune splenocytes in response to stimulation with PmpG-1 peptide, PmpE/F-2₂₅₋₅₇₅ protein, RplF peptide (SEQ ID NO: 10) and RplF protein were strong. The response to the Aasf peptide (SEQ ID NO: 12), Aasf protein or MOMP protein were moderate and others were weaker. Thus, three of the eight antigens (PmpG-1, RplF and PmpE/F-2 - SEQ ID NO: 10, 13 and 16) were determined as immunodominant based on their strong IFN-gamma responses by ELISPOT assay to stimulation by either the peptide or parent protein.

### Example 6: Efficient intracellular uptake of Chlamydia protein antigens by DCs using DOTAP as a delivery system

Since protein antigens require endocytotosis and lysosomal processing before the peptide is loaded onto MHC class II molecules, the cationic liposome DOTAP was used to deliver the *Chlamydia* proteins intracellularly into DCs. The intracellular uptake of PmpG-1₂₅₋₅₀₀, PmpE/F-2₂₅₋₅₇₅, RplF or MOMP protein was visualized by immunohistochemistry following transfection (data not shown). Efficient uptake of PmpG-1₂₅₋₅₀₀, RplF, PmpE/F-2₂₅₋₅₇₅ and MOMP was detected in the cytoplasm of the *Chlamydia* protein-transfected DC, whereas no signal was detected in non-transfected DCs. Thus the cationic liposome DOTAP efficiently delivered *Chlamydia* protein intracellularly into DCs.

After DC transfection with *Chlamydia* proteins, DCs were matured with LPS for 18 h. The cell surface antigen expression on the transfected DCs was assessed after LPS stimulation. There was no phenotypic difference between DCs transfected with different *Chlamydia* antigens or GST (data not shown). DCs stimulated with LPS expressed enhanced levels of CD40, MHC class II and CD86 compared with unstimulated DCs. (data not shown).

### Example 7: Specific immune responses to Chlamydia antigens following adoptive transfer of DCs transfected with Chlamydia proteins

Mice were adoptively transferred with DCs that had been previously transfected with the immunodominant protein antigens. A group of DCs transfected with MOMP was set up as a reference control antigen. As a negative control, one group of mice received DCs pulsed with GST protein. As a positive control, another group of mice received DCs pulsed with viable C. *muridarum* EB. Two weeks following the final adoptive transfer, *Chlamydia*-specific immune responses in vaccinated mice were assessed by enumerating antigen-specific IFN-gamma producing cells in splenocytes from each group after exposure to *Chlamydia* antigens (Figure 5). The results showed that the mice which received the DCs transfected with individual *Chlamydia muridarum* proteins (PmpG-1, RplF and PmpE/F-2) developed significant antigen specific IFN-gamma responses to the corresponding peptides and proteins but not to other non-related *Chlamydia* antigens. Importantly, mice immunized with DCs transfected with individual *Chlamydia* proteins demonstrated strong specific immune responses to HK EB (*p* < 0.01). As a positive control, mice that received DCs pulsed with live *C. muridarum* (EB) developed the strongest IFN-gamma responses to HK-EB as shown by more than 1000 IFN-gamma-secreting cells detected among 10⁶ splenocytes. This group also exhibited strong antigen-specific IFN-gamma responses to PmpG-1 peptide (SEQ ID NO: 13) or PmpG-1 protein and RplF peptide (SEQ ID NO: 10) or RplF protein and moderate responses to PmpE/F-2 peptide (SEQ ID NO: 16) or PmpE/F-2 protein and MOMP. In contrast, naive and GST-DC vaccinated splenocytes stimulated with the *Chlamydia* antigens or HK-EB showed low background levels except for the GST-DC group which exhibited some responses to GST protein and the GST-fusion protein, RplF. IL-4 ELISPOT assays were also performed and showed no or very low *Chlamydia* antigen specific IL-4 secretion in any groups immunized with DCs transfected with individual *Chlamydia* protein (data not shown).

### Example 8: Adoptive transfer of DCs transfected with the PmpG-1, PmpE/F-2 or RplF protein antigens leads to protection against Chlamydia infection in mice.

To evaluate whether the *Chlamydia* protein antigens were able to protect mice against subsequent *Chlamydia* pulmonary or genital tract infection, we undertook adoptive transfer studies using LPS-matured DCs transfected *ex vivo* with either PmpG-1₂₅₋₅₀₀, RplF, PmpE/F-2₂₅₋₅₇₅ or MOMP. Mice received DCs transfected with GST or pulsed with viable C. *muridarum* were set up as negative and positive controls, respectively. Two weeks following the final adoptive transfer, mice were challenged intranasally or vaginally with *C. muridarum.*

After the intranasal challenge, protection was measured by body weight loss and bacterial load in the lungs. As shown in Figure 6A, mice adoptively immunized with live EB-pulsed DC demonstrated excellent protection against infection as indicated by no body weight loss. In contrast, mice immunized with GST-pulsed DC exhibited the largest weight loss. The mean body weight loss on day 10 post infection reached 24.4±2.4% in the negative control group (*p* < 0.001 *vs*. positive control). Mice vaccinated with the individual DC that were transfected with individual *Chlamydia muridarum* protein antigens showed varying levels of protection as indicated by different degrees of body weight loss during the 10-day period. The mean relative body weight loss at day 10 in groups of PmpE/F-2-DC, PmpG-1-DC, RplF-DC, or MOMP was 7.9±3.1%, 8.1±2.7%, 15.2±3.4%, and 19.4±2.8% respectively.

Ten days after the intranasal challenge, lungs were harvested and *Chlamydia* inclusion forming units were determined by plating serial dilutions of homogenized lungs onto HeLa 229 cells (Figure 6B). When compared to the negative control group, the median *Chlamydia* titers decreased 1.8 orders of magnitude (log₁₀) in mice vaccinated with PmpG-1-DC (*p* < 0.01) and decreased 1.2 and 1.1 orders of magnitude in mice vaccinated with RplF-DC (*p* < 0.05) and PmpE/F-2-DC (*p* < 0.05) respectively. There was no statistically significant difference in lung *Chlamydia* titers between mice vaccinated with MOMP-DC and the negative control group.

Protection against intravaginal infection was assessed by isolation of *Chlamydia* from cervicovaginal wash and determination of the number of IFU recovered from each experimental group at day 6 post-infection (Figure 7). The results showed that the cervicovaginal shedding of *C*. *muridarum* in mice immunized with any of the four *Chlamydia* protein-transfected DCs was significantly lower than that of mice who received GST-transfected DCs (*p* < 0.001 in PmpG-1 group; *p* < 0.01 in RplF group; *p* < 0.01 in PmpE/F-2 group; *p* < 0.01 in MOMP group). Taken together, mice vaccinated with DCs transfected with *Chlamydia* protein PmpG-1₂₅₋₅₀₀, RplF or PmpE/F-2₂₅₋₅₇₅ polypeptides exhibited significant resistance to challenge infection as indicated by log₁₀ reduction in the median *Chlamydia* titer in comparison with the negative control group in both lung model and genital tract model. MOMP, as a reference antigen, conferred significant protection but only in the genital tract model. These data demonstrated that vaccination with DCs transfected with PmpG-1₂₅₋₅₀₀ polypeptide developed the greatest degree of protective immunity among the four *Chlamydia* antigens evaluated.

### Example 9: Vaccination with both PmpG-1 and PmpE/F-2 protein antigens leads to synergistic protection against Chlamydia infection in mice

To evaluate whether combinations of *Chlamydia* protein antigens were able to protect mice against genital tract infection, we vaccinated mice with either PmpG-1₂₅₋₅₀₀, PmpE/F-2₂₅₋₅₇₅ or MOMP, or a pool of PmpG-1₂₅₋₅₀₀, PmpE/F-2₂₅₋₅₇₅ and MOMP, formulated with adjuvant DDA/TDB. C57BL/6 mice were vaccinated three times with a 2-week interval with PBS, DDA/TDB alone as negative controls and live *Chlamydia* EB as positive control. One week after the final immunization, the mice from each group were injected with Depo-Provera. One week after Depo-Provera treatment, the mice were infected intravaginally with 1500 IFU live *C. muridarum.* Protection against intravaginal infection was assessed by isolation of *Chlamydia* from cervicovaginal wash and determination of the number of IFU recovered from each experimental group at day 6 and day 13 post-infection (Figure 8).

As shown in Figure 8, mice immunized with EB demonstrated excellent protection against infection as indicated by large reductions in cervicovaginal shedding at 6 and 13 days post infection. In contrast, the negative control (DDA/TDB adjuvant alone) group of mice, showed very high levels of cervicovaginal shedding. When compared to the negative control group, the median cervicovaginal shedding decreased 1.0and 2.9 orders of magnitude (log₁₀) in mice vaccinated with PmpG-1 (*p* < 0.01, p<0.001) on day 6 and day 13. The bacterial titer decreased 0.8 and 1.1 orders of magnitude in mice vaccinated with PmpE/F-2 (*p*< 0.05, p<0.05). Cervicovaginal shedding decreased 1.8 and 3.8 orders of magnitude in mice vaccinated with a cocktail containingPmpG-1 PmpE/F-2 and MOMP (p <0.01, p<0.001). MOMP, as a reference antigen, conferred significant protection at both 6 and 13 days post infection. Taken together, mice vaccinated with *Chlamydia* protein PmpG-1₂₅₋₅₀₀ and PmpE/F-2₂₅₋₅₇₅ exhibited significant resistance to challenge infection as indicated by reduction in the median *Chlamydia* titer in comparison with the adjuvant alone group in the genital tract model.

### Example 10: Multiple Chlamydia antigens formulated with DDA/TDB exhibit protection against challenge with live C. muridarum

In order to discover a Th1-polarizing adjuvant that efficiently delivers *Chlamydia* antigens, we first tested mouse specific CpG-ODN 1826. In the current trial, mice were immunized with PmpG-1 or MOMP protein formulated with either a free form of CpG ODN 1826 (Free CpG) or a liposomal nanoparticle conjugated form (LN-CpG). Mice immunized with PmpG-1 plus liposomal nanoparticle only (PmpG+LN), LN-CpG only or PBS were set up as negative controls and mice recovered from previous intranasal infection served as a positive control. Two weeks after the final immunization, mice were challenged vaginally with *C. muridarum.* Protection against intravaginal infection was assessed by isolation of *Chlamydia* from cervicovaginal wash and the determination of the number of IFU recovered from each experimental group at day 6 post-infection. As shown in Fig.9a, mice immunized with live EB exhibited excellent protection against infection, as indicated by no or very low *Chlamydia* detection. However, the cervicovaginal shedding of *C. muridarum* in all other groups did not have any significant difference (Fig. 9a), demonstrating that CpG ODN formulated with PmpG-1 or MOMP failed to induce protection against *Chlamydia* infection.

In the next trial we evaluated protection against *Chlamydia* infection in C57 mice immunized with individual PmpG-1, PmpE/F-2, MOMP protein or a combination formulated with adjuvant AbISCO-100 or DDA/TDB. After the genital challenge, we tested the *Chlamydia* inclusion titers in cervicovaginal washes taken at day 6 and day 13. The results indicate that DDA/TDB exhibited overall better protection than AbISCO. As shown in Fig.9b, mice immunized with individual PmpG-1, PmpE/F-2, MOMP protein or a combination formulated with DDA/TDB demonstrated significant reduction of *Chlamydia* titer at day 6 when compared to DDA/TDB adjuvant alone group (*p* < 0.01 in the PmpG+DDA/TDB group, *p* < 0.05 in the PmpF+DDA/TDB group, *p* < 0.01 in the MOMP+DDA/TDB group and *p* < 0.01 in the G+F+M+DDA/TDB group). The antigen combination group tended to develop higher protection than individual antigen group, as indicated by much lower *Chlamydia* titers detected in some mice of the G+F+M+DDA/TDB group. Significant protection induced by AbISCO was only observed in the combination group (*p* < 0.01 vs AbISCO alone), but not in the individual antigen group. Data at day 13 (Fig.9c) further confirmed the results obtained at day 6. Mice immunized with individual PmpG-1 protein or the combination of three *Chlamydia* proteins formulated with AbISCO exhibited significant protection at day 13 compared to the adjuvant alone group (*p* < 0.05 in the PmpG+AbISCO group and *p* < 0.01 in the G+F+M+ AbISCO group). On the other hand, all DDA/TDB formulated- *Chlamydia* antigens conferred significant protection at day 13 when compared to DDA/TDA alone group and vaccination with G+F+M+DDA/TDB exhibited the greatest degree of protective immunity among all the groups tested. Of interest, five out of eight mice vaccinated with G+F+M+DDA/TDB completely resolved the infection and the other three mice in this group showed very low *Chlamydia* load at day 13 (*p* < 0.01 in the PmpG+DDA/TDB group, *p* < 0.05 in the PmpF+DDA/TDB group, *p* < 0.05 in the MOMP+DDA/TDB group and *p* < 0.001 in the G+F+M+DDA/TDB group).

Since all the protection results obtained above were observed in C57BL/6 mouse, the strain in which the antigens were originally discovered by immunoproteomics, we challenged mice with a different MHC genetic background to determine if immunization with multiple *Chlamydia* protein antigens and DDA/TDB conferred protection. BALB/c mice were immunized with G+F+M+DDA/TDB or DDA/TDB alone, and mice infected with live EB were set up as a positive control. *Chlamydia* inclusion titers in the cervicovaginal washes were detected post-challenge. As shown in Fig. 9d, BALB/c mice immunized with live EB demonstrated excellent protection against infection, as indicated by very low bacterial load at day 6, and no *Chlamydia* detected at day 13 and day 20.Vaccination with G+F+M+DDA/TDB in BALB/c mice significantly decreased the *Chlamydia* load in the cervicovaginal washes at all three selected dates when compared with DDA/TDB alone (*p* < 0.001). At day 20 after challenge, all BALB/c mice vaccinated with G+F+M+DDA/TDB completely resolved infection.

Collectively, among the three tested adjuvants CpG ODN 1826, AbISCO-100 and DDA/TDB, CpG ODN formulation was not able to engender protection against *Chlamydia* infection at any level in vaccinated mice. The AbISCO formulation conferred moderate protection while the DDA/TDB formulation showed the greatest efficacy. The combination of PmpG-1, PmpE/F-2 and MOMP formulated with DDA/TDB generated a synergistic effect that exhibited the greatest degree of protective immunity among all groups studied. Moreover, G+F+M+DDA/TDB vaccination also stimulated significant protection in BALB/c mice with a different MHC background from C57BL/6 mice.

### Example 11: PmpG-1 formulated with DDA/TDB induced strong IFN-γ, TNF-α and IL-17 responses characterized by a high frequency of IFN-γ/ TNF-α and IFN-γ/IL-17 double positive CD4+ T cells in immunized mice.

In order to explore the cellular mechanisms for different degrees of protection induced by the three adjuvants, C57BL/6 mice were immunized with PmpG-1 formulated with DDA/TDB, AbISCO-100 and CpG ODN 1826 and then challenged with live *C. muridarum.* The magnitude and quality of T cells producing IFN-γ, TNF-α and IL-17 were assessed before and after challenge using ELISPOTs, ELISA and multiparameter flow cytometry.

In this study, the ELISPOTs assay was performed to detect IFN-γ and IL-17 producing cells in immune splenocytes stimulated with PmpG-1 protein or HK-EB. ELISA was performed to measure TNF-α level in the supernatant of stimulated immune splenocytes. Splenocytes after immunization with PmpG-1 formulated with DDA/TDB, AbISCO-100 or CpG ODN 1826 exhibited markedly different levels of IFN-γ (Fig. 10a), TNF-α (Fig. 10c) and IL-17 response (Fig. 10b). The PmpG+DDA/TDB immune splenocytes exposed to either PmpG-1 protein or HK-EB developed the highest numbers of IFN-γ, and IL-17-secreting cells; the PmpG+AbISCO immune splenocytes demonstrated less strong IFN-γ and IL-17 responses but similar levels of TNF-α when compared with PmpG+DDA/TDB immunization; and the weakest IFN-γ, TNF-α response and no IL-17 response were induced by the PmpG+CpG immunization. In addition, splenocytes from adjuvant alone immunized mice which served as negative controls showed nearly blank background levels, indicating that cytokine responses detected in the experimental system are *Chlamydia* Ag-specific. The varying levels of IFN-γ and IL-17 response in mice immunized with different adjuvants are remarkably consistent with the degree of protection against challenge infection (Fig 9) suggesting that a correlate of vaccine-mediated protection against *Chlamydia* is the magnitude of specific cytokine responses.

To characterize the distinct populations of Th1 and Th17 responses, multiparameter flow cytometry was used to simultaneously analyze multiple cytokines at the single-cell level. As shown in Fig11a, a seven-color flow cytometry panel and gating strategy was used to identify IFN-gamma, TNF-alpha and IL-17 producing CD4+ T cells in splenocytes from a representative mouse immunized with PmpG+DDA/TDB. Since an individual responding cell could be present in more than one of the total cytokine gates, we used Boolean combinations of the cytokine gates to discriminate responding cells based on their functionality or quality of IFN-γ/TNF-α (Fig. 11b) and IFN-γ/IL-17 (Fig. 11c) production.

Using the Boolean combination of IFN-γ or TNF-α gate, frequencies of three distinct populations (IFN-γ+TNF-α-, IFN-γ-TNF-α+, IFN-γ+TNF-α+) from immune splenocytes stimulated with PmpG-1 and HK-EB are shown in Fig.11b-1 and Fig.11b-2 respectively. The results demonstrate that the response after immunization with PmpG+DDA/TDB was dominated by IFN-γ and TNF-α double positive cells and about half of the response in the PmpG+AbISCO group was IFN-γ and TNF-α+ double positive, whereas the PmpG+CpG vaccine induced the weakest IFN-γ and TNF-α+ double positive response and the single positive dominate response. Importantly, the analysis showed a correlation between the frequency of multifunctional (IFN-γ, TNF-α double-positive) CD4+ T cells and the degree of protection in mice vaccinated with PmpG+DDA/TDB, PmpG+AbISCO and PmpG+CpG. In this study, the quality of IFN-γ/IL-17 cytokine response from immune splenocytes stimulated with PmpG (Fig. 11c-1) or HK-EB (Fig. 11c-2) was evaluated by multiparameter flow cytometry. Quantifying the fraction of IFN-γ/IL-17 response, we found that over half of the response in the most protected group (PmpG+DDA/TDB) was IFN-γ and IL-17 double positive; the PmpG+AbISCO group induced a moderate IFN-γ and IL-17 double positive response. The no protection group (PmpG+CpG) did not develop a measurable IL-17 response. The data indicate a correlation between the degree of protection in the vaccinated mice and the frequency of IFN-γ and IL-17 double positive CD4+ T cells as well as IFN-γ and TNF-α double positive CD4+ T cells.

### Example 12: The magnitude and quality of IFN-γ, TNF-α and IL-17 responses in spleens and lymph nodes after challenge.

To define the magnitude of the response on day 7 after *C. muridarum* challenge, the frequency of the total PmpG-specific CD4+ T cell cytokine responses comprising IFN-γ, TNF-α and IL-17 producing cells in spleen (Fig. 12a) and draining lymph node (iliac lymph node) (Fig. 12b) are presented from each vaccine group. The results demonstrate among spleen cells that immunization with PmpG+DDA/TDB induced the highest frequency of IFN-γ and IL-17 producing CD4+ T cells; the PmpG+AbISCO group induced a similar frequency of TNF-α producing cell but a lower frequency of IFN-γ and IL-17 producing cells when compared with PmpG+DDA/TDB group; PmpG+CpG and PBS group developed similar but lowest frequency of IFN-γ and TNF-α producing cells. Notably, PmpG+CpG group did not induce a measurable IL-17 response while the PBS group demonstrated about one third of the magnitude for the IL-17 response compared with PmpG+DDA/TDB group (Fig.12a). Shown is the mean± SEM (n=3 or 4) for one of at least two experiments.

The data from pooled regional draining lymph node cells following genital challenge showed that prior immunization with PmpG+DDA/TDB resulted in strong IFN-γ and TNF-α responses. The PmpG+AbISCO and PBS groups developed similar moderate IFN-γ and TNF-α responses. The PmpG+CpG group induced the weakest IFN-γ and TNF-α responses. Surprisingly, and contrary to the spleen cell results, the IL-17 response in lymph node was very low in the PmpG+DDA/TDB and PmpG+AbISCO group, and no IL-17 producing cells were observed in the PmpG+CpG and PBS group (Fig. 12b).

We further analyzed the quality of cytokine producing cells in spleen and iliac lymph node from immunized mice following genital challenge. Immunization with PmpG+DDA/TDB developed the strongest IFN-γ and TNF-α double positive response in both spleen (Fig. 12c-1) and lymph node (Fig. 12c-2). Immunization with PmpG+AbISCO induced moderate IFN-γ and TNF-α double positive response. We found very few or no IFN-γ and TNF-α double positive response cells in the PmpG+CpG group and in the PBS group (Fig. 12c-1&12c-2). Analysis of the IFN-γ/IL-17 response in spleen (Fig. 12d-1) after challenge in the three PmpG vaccine groups exhibited the strongest IFN-γ and IL-17 double positive response in PmpG+DDA/TDB group, moderate response in PmpG+AbISCO group and the weakest in PmpG+CpG. These findings show a similar pattern as before challenge (Fig. 11). However, low IL-17 producing cells, especially few IFN-γ/IL-17 double positive cells, were detected in the lymph node (Fig.12d-2) after challenge. Notably, despite the PBS group developing IFN-γ, TNF-α and IL-17 responses after challenge, we observed that all three cytokine producing cells in this group were single positive in both spleen and lymph node (Fig. 12c and Fig. 12d). These data further confirm our findings demonstrating a connection between the level of protection and the magnitude and quality of IFN-γ, IL-17 and TNF-α production.

### Example 13: Pathologic changes

We evaluated the effect of immunization of the *Chlamydia muridarum* antigen combination on inflammatory pathology in C57BL/6 mouse upper genital tract following *Chlamydia muridarum* infection. Sixty days after the intravaginal challenge infection, mice were sacrificed and mouse genital tract tissues were collected for pathology observation. The genital tract tissues from mice immunized with G+F+M+DDA/TDB, G+F+M+AbISCO, PBS or live EB were examined at the level of gross appearance (G+F+M = PmpG-1, PmpE/F-2 and MOMP pooled). Hydrosalpinx is a visual hallmark of inflammatory pathology in the fallopian tube induced by *Chlamydia muridarum* infection. Six of 8 mice in PBS group developed obvious hydrosalpinx in either one or both fallopian tubes (3 mice bilateral, 3 mice unilateral). Six of the 8 mice vaccinated with G+F+M+DDA/TDB (2 mice bilateral, 4 mice unilateral) and eight of the 8 mice vaccinated with G+F+M+AbISCO (4 mice bilateral, 4 mice unilateral) had hydrosalpinx. The pathologic outcome in both G+F+M+DDA/TDB and G+F+M+AbISCO groups was not significantly different from that in PBS group. Mice recovered from a prior intranasal infection were however completely protected against the development of oviductal hydrosalpinx pathology.

### Example 14: Induction of CD4+ T cells by C.trachomatis epitopes.

C57 BL/6 mice were immunized three times subcutaneously in the base of tail with a cocktail of C. *trachomatis* serovar D polypeptides PmpG (SEQ ID NO: 42), PmpF (SEQ IDN O: 43) and MOMP (SEQ IDNO: 44), formulated with DDA/TDB adjuvant (G+F+M+DDA/TDB) at 2-week intervals. Adjuvant alone (DDA/TDB) was administered as control. Two weeks after the final immunization, splenocytes were harvested and stimulated with 1 microgram/ml *C. trachomatis* serovar D protein PmpG, PmpF, MOMP or 5X10⁵ inclusion-forming units (IFU) /ml heat-killed EB respectively. DDA/TDB alone adjuvant was set up as a negative control. Interferon gamma response in mice was determined by an ELISPOT assay (Figure 13). The results represent the average of duplicate wells and are expressed as means ± SEM for groups of six mice.

These studies demonstrate that CD4+ T cells of mice immunized with a C. *trachomatis* antigen composition can be stimulated by individual components of the antigen composition and produce IFN-gamma.

One or more currently preferred embodiments of the invention have been described by way of example. The invention includes all embodiments, modifications and variations substantially as hereinbefore described and with reference to the examples and figures. It will be apparent to persons skilled in the art that a number of variations and modifications can be made without departing from the scope of the invention as defined in the claims. Examples of such modifications include the substitution of known equivalents for any aspect of the invention in order to achieve the same result in substantially the same way.

## Claims

1. A composition comprising a PmpG, PmpF, and major outer membrane protein 1 (MOMP) polypeptide in combination with an adjuvant, wherein the PmpG polypeptide comprises an amino acid sequence set forth in SEQ ID NO: 13, SEQ ID NO: 42, or SEQ ID NO: 45; the PmpF polypeptide comprises an amino acid sequence set forth in SEQ ID NO: 16, SEQ ID NO: 43, or SEQ ID NO: 46; and the MOMP polypeptide comprises an amino acid sequence set forth in SEQ ID NO: 44 or SEQ ID NO: 47.

2. The composition of claim 1 wherein the PmpG polypeptide consists of an amino acid sequence set forth in SEQ ID NO: 13, SEQ ID NO: 42, or SEQ ID NO: 45; the PmpF polypeptide consists of an amino acid sequence set forth in SEQ ID NO: 16, SEQ ID NO: 43, or SEQ ID NO: 46; and the MOMP polypeptide consists of an amino acid sequence set forth in SEQ ID NO: 44 or SEQ ID NO: 47.

3. The composition of claim 1 or 2 wherein the polypeptides are *Chlamydia trachomatis* polypeptides or *Chlamydia muridarum* polypeptides.

4. The composition of claim 1 or 2 wherein the PmpG polypeptide comprises an amino acid sequence set forth in SEQ ID NO: 42.

5. The composition of claim 1 or 2 wherein the PmpF polypeptide comprises an amino acid sequence set forth in SEQ ID NO: 43.

6. The composition of claim 1 or 2 wherein the MOMP polypeptide comprises an amino acid sequence set forth in SEQ ID NO: 44.

7. The composition of any one of claims 1 to 6, wherein the adjuvant is dimethyldioctadecylammonium bromide and trehalose 6,6'-dibehenate (DDA/TDB) or AbISCO.

8. The composition of any one of claims 1 to 7, wherein the PmpG polypeptide comprises an amino acid sequence set forth in SEQ ID NO: 42; the PmpF polypeptide comprises an amino acid sequence set forth in SEQ ID NO: 43; the MOMP polypeptide comprises an amino acid sequence set forth in SEQ ID NO: 44; and the adjuvant is dimethyldioctadecylammonium bromide and trehalose 6,6'-dibehenate (DDA/TDB).

9. The composition of any one of claims 1 to 8, wherein the composition induces an immune response to a *Chlamydia* species in a subject.

10. The composition of claim 9 wherein the immune response is a cellular immune response.

11. The composition of claim 9 or 10 wherein the *Chlamydia* species is *C. trachomatis* or *C. muridarum.*

12. The composition of any one of claims 1 to 11 for use in the treatment or prevention of a *Chlamydia* infection in a subject.

13. The composition for use of claim 12 wherein the *Chlamydia* infection is in a lung or genital tract.

14. The composition for use of claim 12 or 13 wherein the *Chlamydia* infection is associated with *C. trachomatis.*

15. The composition for use of any one of claims 12 to 14 wherein the composition is for intranasal administration or for injection.

16. The composition or composition for use of any one of claims 9 to 15 wherein the subject is a human.

## Patentansprüche

1. Zusammensetzung, umfassend ein PmpG-, PmpF- und Hauptaußenmembranprotein 1 (MOMP)-Polypeptid in Kombination mit einem Adjuvans, wobei das PmpG-Polypeptid eine in SEQ ID NR.: 13, SEQ ID NR.: 42 oder SEQ ID NR.: 45 angeführte Aminosäuresequenz umfasst; das PmpF-Polypeptid eine in SEQ ID NR.: 16, SEQ ID NR.: 43 oder SEQ ID NR.: 46 angeführte Aminosäuresequenz umfasst; und das MOMP-Polypeptid eine in SEQ ID NR.: 44 oder SEQ ID NR.: 47 angeführte Aminosäuresequenz umfasst.

2. Zusammensetzung nach Anspruch 1, wobei das PmpG-Polypeptid aus einer in SEQ ID NR.: 13, SEQ ID NR.: 42 oder SEQ ID NR.: 45 angeführten Aminosäuresequenz besteht; das PmpF-Polypeptid aus einer in SEQ ID NR.: 16, SEQ ID NR.: 43 oder SEQ ID NR.: 46 angeführten Aminosäuresequenz besteht; und das MOMP-Polypeptid aus einer in SEQ ID NR.: 44 oder SEQ ID NR.: 47 angeführten Aminosäuresequenz besteht.

3. Zusammensetzung nach Anspruch 1 oder 2, wobei die Polypeptide *Chlamydia trachomatis-*Polypeptide oder *Chlamydia muridarum-*Polypeptide sind.

4. Zusammensetzung nach Anspruch 1 oder 2, wobei das PmpG-Polypeptid eine in SEQ ID NR.: 42 angeführte Aminosäuresequenz umfasst.

5. Zusammensetzung nach Anspruch 1 oder 2, wobei das PmpF-Polypeptid eine in SEQ ID NR.: 43 angeführte Aminosäuresequenz umfasst.

6. Zusammensetzung nach Anspruch 1 oder 2, wobei das MOMP-Polypeptid eine in SEQ ID NR.: 44 angeführte Aminosäuresequenz umfasst.

7. Zusammensetzung nach einem der Ansprüche 1 bis 6, wobei das Adjuvans Dimethyldioctadecylammoniumbromid und Trehalose-6,6'-dibehenat (DDA/TDB) oder AbISCO ist.

8. Zusammensetzung nach einem der Ansprüche 1 bis 7, wobei das PmpG-Polypeptid eine in SEQ ID NR.: 42 angeführte Aminosäuresequenz umfasst; das PmpF-Polypeptid eine in SEQ ID NR.: 43 angeführte Aminosäuresequenz umfasst; das MOMP-Polypeptid eine in SEQ ID NR.: 44 angeführte Aminosäuresequenz umfasst; und das Adjuvans Dimethyldioctadecylammoniumbromid und Trehalose-6,6'-dibehenat (DDA/TDB) ist.

9. Zusammensetzung nach einem der Ansprüche 1 bis 8, wobei die Zusammensetzung eine Immunreaktion auf eine *Chlamydien-*Spezies in einem Subjekt einleitet.

10. Zusammensetzung nach Anspruch 9, wobei die Immunreaktion eine zelluläre Immunreaktion ist.

11. Zusammensetzung nach Anspruch 9 oder 10, wobei die *Chlamydien*-Spezies *C. trachomatis* oder *C. muridarum* ist.

12. Zusammensetzung nach einem der Ansprüche 1 bis 11 zur Verwendung bei der Behandlung oder Prävention einer *Chlamydien-*Infektion bei einem Subjekt.

13. Zusammensetzung zur Verwendung nach Anspruch 12, wobei die *Chlamydien-*Infektion in einer Lunge oder einem Genitaltrakt ist.

14. Zusammensetzung zur Verwendung nach Anspruch 12 oder 13, wobei die *Chlamydien-*Infektion mit *C. trachomatis* verbunden ist.

15. Zusammensetzung zur Verwendung nach einem der Ansprüche 12 bis 14, wobei die Zusammensetzung zur intranasalen Verabreichung oder zur Injektion ist.

16. Zusammensetzung oder Zusammensetzung zur Verwendung nach einem der Ansprüche 9 bis 15, wobei das Subjekt ein Mensch ist.

## Revendications

1. Composition comprenant un polypeptide PmpG, PmpF, et un polypeptide majeur de la protéine de membrane externe 1 (MOMP) en combinaison avec un adjuvant, dans laquelle le polypeptide PmpG comprend une séquence d'acides aminés représentée par SEQ ID NO: 13, SEQ ID NO: 42 ou SEQ ID NO: 45; le polypeptide PmpF comprend une séquence d'acides aminés représentée par SEQ ID NO: 16, SEQ ID NO: 43 ou SEQ ID NO: 46; et le polypeptide MOMP comprend une séquence d'acides aminés représentée dans SEQ ID NO: 44 ou SEQ ID NO: 47.

2. Composition selon la revendication 1, dans laquelle le polypeptide PmpG consiste en une séquence d'acides aminés représentée par SEQ ID NO: 13, SEQ ID NO: 42, ou SEQ ID NO: 45; le polypeptide PmpF consiste en une séquence d'acides aminés présentée par SEQ ID NO: 16, SEQ ID NO: 43, ou SEQ ID NO: 46; et le polypeptide MOMP consiste en une séquence d'acides aminés représentée par SEQ ID NO: 44 ou SEQ ID NO: 47.

3. Composition selon la revendication 1 ou 2, dans laquelle les polypeptides sont des polypeptides de *Chlamydia trachomatis* ou des polypeptides de *Chlamydia muridarum.*

4. Composition selon la revendication 1 ou 2, dans laquelle le polypeptide PmpG comprend une séquence d'acides aminés représentée par SEQ ID NO: 42.

5. Composition selon la revendication 1 ou 2, dans laquelle le polypeptide PmpF comprend une séquence d'acides aminés représentée par SEQ ID NO: 43.

6. Composition selon la revendication 1 ou 2, dans laquelle le polypeptide MOMP comprend une séquence d'acides aminés représentée dans SEQ ID NO: 44.

7. Composition selon l'une quelconque des revendications 1 à 6, dans laquelle l'adjuvant est le bromure de diméthyldioctadécylammonium et le tréhalose-6,6'-dibéhénate (DDA/TDB) ou AbISCO.

8. Composition selon l'une quelconque des revendications 1 à 7, dans laquelle le polypeptide PmpG comprend une séquence d'acides aminés représentée par SEQ ID NO: 42; le polypeptide PmpF comprend une séquence d'acides aminés représentée dans SEQ ID NO: 43; le polypeptide MOMP comprend une séquence d'acides aminés représentée par SEQ ID NO: 44; et l'adjuvant est le bromure de diméthyldioctadécylammonium et le tréhalose-6,6'-dibéhénate (DDA/TDB).

9. Composition selon l'une quelconque des revendications 1 à 8, dans laquelle la composition induit une réponse immunitaire à une espèce de *Chlamydia* chez un sujet.

10. Composition selon la revendication 9 dans laquelle la réponse immunitaire est une réponse immunitaire cellulaire.

11. Composition selon la revendication 9 ou 10 dans laquelle l'espèce *Chlamydia* est *C. trachomatis* ou *C. muridarum.*

12. Composition selon l'une quelconque des revendications 1 à 11 pour une utilisation dans le traitement ou la prévention d'une infection à *Chlamydia* chez un sujet.

13. Composition pour une utilisation selon la revendication 12 dans laquelle l'infection à *Chlamydia* est dans un poumon ou un tractus génital.

14. Composition pour une utilisation selon la revendication 12 ou 13 dans laquelle l'infection à *Chlamydia* est associée à C. *trachomatis.*

15. Composition pour une utilisation selon l'une quelconque des revendications 12 à 14 dans laquelle la composition est destinée à une administration intranasale ou à une injection.

16. Composition pour une utilisation selon l'une quelconque des revendications 9 à 15 dans laquelle le sujet est un humain.
